# EUROPÄISCHE PATENTSCHRIFT

(11) **EP 0 726 270 B1**
(45) Veröffentlichungstag und Bekanntmachung des Hinweises auf die Patenterteilung: **23.05.2001**
(21) Anmeldenummer: 96101170.7
(22) Anmeldetag: 29.01.1996
(51) Int. Cl.: C07D 471/04, C07D 519/00, C07D 498/16, A61K 31/435, A61K 31/535, C07D 213/78

(54) **1,6-Naphthyridoncarbonsäurederivate**
Derivatives of 1,6-naphthyridone carboxylic acids
Dérivés d'acides carboxyliques de la 1,6-naphthyridone

(30) Priorität: 09.02.1995 DE 19504280; 24.02.1995 DE 19506535
(43) Veröffentlichungstag der Anmeldung: 14.08.1996
(73) Patentinhaber: BAYER AG, 51368 Leverkusen (DE)
(72) Erfinder: Bartel, Stephan, Dr., D-51465 Bergisch Gladbach (DE); Grohe, Klaus, Dr., D-51519 Odenthal (DE); Hagemann, Hermann, Dr., D-51375 Leverkusen (DE); Bremm, Klaus-Dieter, Dr., D-45661 Recklinghausen (DE); Endermann, Rainer, Dr., D-42113 Wuppertal (DE)

(56) Entgegenhaltungen:
- EP-A- 0 588 166
- EP-A- 0 607 825
- DE-A- 2 362 553
- DE-A- 3 033 157
- US-A- 3 637 716
- CHEMICAL ABSTRACTS, vol. 90, no. 13, 1979 Columbus, Ohio, US; abstract no. 103851r, SHIBANOV ET AL.: "2,4,6-Trichloronicotinic acid or its chloride as intermediate for the synthesis of biologically active compounds" Seite 583; XP002003058 & SU-A-638 595 (SHIBANOV)
- CHIMICA THERAPEUTICA, Nr. 12, 1977, PARIS FR, Seiten 541-547, XP002003057 STREHLKE: "Untersuchungen über die antibakterielle Aktivität von Chinoloncarbonsäauren"

## Beschreibung

Die Erfindung betrifft neue 1,6-Naphthyridoncarbonsäurederivate, Verfahren zu ihrer Herstellung, diese enthaltende antibakterielle Mittel und Futterzusatzstoffe.

Es ist bereits bekannt geworden, daß derartige 1,6-Naphthyridoncarbonsäuren antibakteriell wirksam sind. Beispiele hierfür finden sich in DE 3033157 A 1, JP 50111096, JP 50111080, JP 50108294, JP 50108277, JP 50108276, JP 50076093, JP 50106974 und DE 2362553. 8-fluorsubstituierte 1,6-Naphthyridone wurden im Journal of Heterocyclic Chemistry **30** (1993), 855 beschrieben. Die EP-A-0588 166 und EP-A-0607 825 offenbaren 7-Isoindolinyl-chinolon- und naphthyridon-Derivate.
Es wurden nun Verbindungen der allgemeinen Formel (I) gefunden 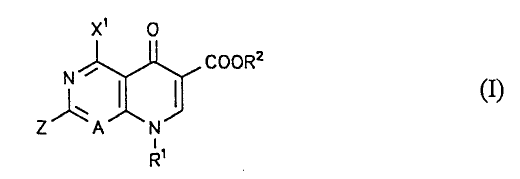 in welcher
- R¹: für gegebenenfalls durch Hydroxy, Halogen oder C₁-C₃-Alkoxy substituiertes geradkettiges oder verzweigtes C₁-C₄-Alkyl, für gegebenenfalls durch C₁-C₃-Alkyl oder Halogen substituiertes C₃-C₆-Cycloalkyl, C₂-C₄-Alkenyl, ferner für C₁-C₃-Alkoxy, Amino, Monoalkylamino mit 1 bis 3 C-Atomen, Dialkylamino mit 1 bis 3 C-Atomen je Alkylgruppe oder für gegebenenfalls durch Halogen ein- bis dreifach substituiertes Phenyl steht,
- R²: für Wasserstoff, gegebenenfalls durch Hydroxy, Methoxy, Amino, Methylamino, oder Dimethylamino substituiertes Alkyl mit 1 bis 4 Kohlenstoffatomen oder (5-Methyl-2-oxo-1,3-dioxol-4-yl)-methyl,
- X¹: für Wasserstoff, Halogen, Amino, Methyl oder Trifluormethyl,
- Z: für Reste der Strukturen 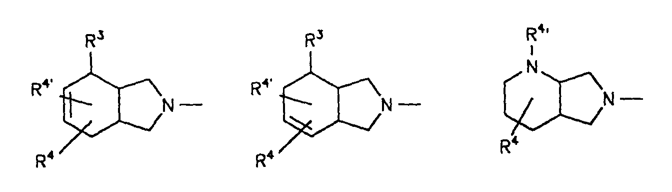 worin
R³ für Wasserstoff, Hydroxy, -NR⁵R⁶, Hydroxymethyl oder -CH₂-NR⁵R⁶ steht,
wobei
R⁵ Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl und
R⁶ Wasserstoff oder Methyl bedeutet,
R⁴ für Wasserstoff, geradkettiges oder verzweigtes C₁-C₃-Alkyl oder Cyclopropyl,
R^{4'} für Wasserstoff oder Methyl,
- A: für C-R⁷ steht, worin
R⁷ für Wasserstoff, Halogen, Methyl, Trifluormethyl, Vinyl, Ethinyl, Hydroxy oder Methoxy steht oder auch gemeinsam mit R¹ eine Brücke der Struktur 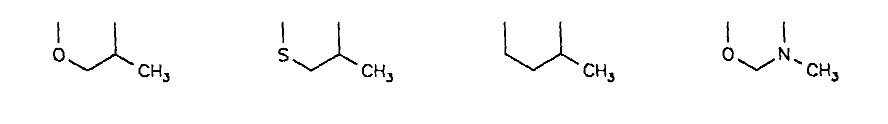
bilden kann, in Form von Racematen oder als enantiomerenreine Verbindungen, deren pharmazeutisch verwendbare Hydrate und Säureadditionsalze sowie die Alkali-, Erdalkali-, Silber- und Guanidiniumsalze der zugrundeliegenden Naphthyridoncarbonsäuren. Die erfindungsgemäßen Verbindungen weisen im Vergleich zu bekannten Vertretern dieses Strukturtypes eine höhere antibakterielle Wirkung insbesondere im grampositiven Bereich auf.

Sie eignen sich daher als Wirkstoffe für die Human- und Veterinärmedizin, wobei zur Veterinärmedizin auch die Behandlung von Fischen zur Therapie oder zur Vorbeugung bakterieller Infektionen zu zählen ist.

Bevorzugt sind Verbindungen der Formel (I), in welcher
- R¹: für gegebenenfalls durch Hydroxy oder Fluor substituiertes C₁-C₂-Alkyl, für gegebenenfalls durch Fluor substituiertes C₃-C₅-Cycloalkyl, Vinyl, Amino, Monoalkylamino mit 1 bis 2 C-Atomen, Dialkylamino mit 1 bis 2 C-Atomen je Alkylgruppe oder für gegebenenfalls durch Halogen ein- bis zweifach substituiertes Phenyl steht,
- R²: für Wasserstoff, gegebenenfalls durch Amino oder Dimethylamino substituiertes Alkyl mit 1 bis 2 Kohlenstoffatomen oder (5-Methyl-2-oxo-1,3-dioxol-4-yl)-methyl,
- X¹: für Wasserstoff, Fluor, Chlor, Amino, Methyl oder Trifluormethyl,
- Z: für Reste der Strukturen 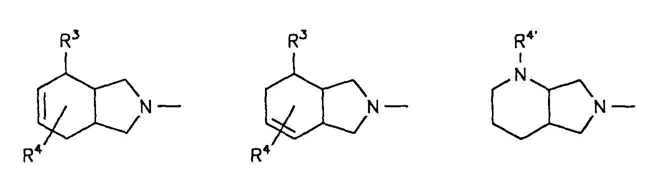 worin
R³ für Wasserstoff, Hydroxy, -NR⁵R⁶, Hydroxymethyl oder -CH₂-NR⁵R⁶ steht,
wobei
R⁵ Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl und
R⁶ Wasserstoff oder Methyl bedeutet,
R⁴ für Wasserstoff, geradkettiges oder verzweigtes C₁-C₃-Alkyl oder Cyclopropyl,
R^{4'} für Wasserstoff oder Methyl,
- A: für C-R⁷ steht, worin
R⁷ für Wasserstoff, Chlor, Fluor, Methyl, Trifluormethyl, Hydroxy oder Methoxy steht oder auch gemeinsam mit R¹ eine Brücke der Struktur 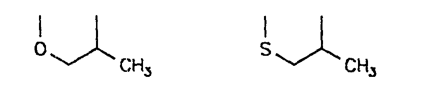 bilden kann,
und deren pharmazeutisch verwendbare Hydrate und Säureadditionsalze sowie die Alkali-, Erdalkali-, Silber- und Guanidiniumsalze der zugrundeliegenden Naphthyridoncarbonsäuren.

Besonders bevorzugt sind die Verbindungen der Formel (I), in welcher
- R¹: für Methyl, Ethyl, gegebenenfalls durch Fluor substituiertes Cyclopropyl oder für gegebenenfalls durch Fluor ein- bis zweifach substituiertes Phenyl steht,
- R²: für Wasserstoff, Methyl oder Ethyl,
- X¹: für Wasserstoff, Methyl oder Trifluormethyl,
- Z: für Reste der Strukturen 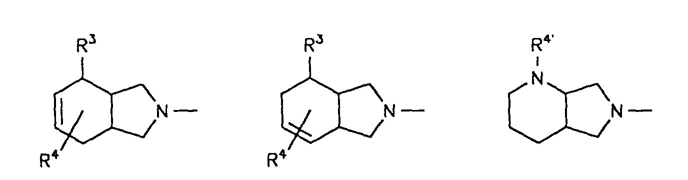 worin
R³ für Wasserstoff, Hydroxy, -NR⁵R⁶, Hydroxymethyl oder -CH₂-NR⁵R⁶ steht,
wobei
R⁵ Wasserstoff, Methyl, Alkoxycarbonyl mit 1 bis 4 C-Atomen im Alkoxyteil oder C₁-C₃-Acyl und
R⁶ Wasserstoff oder Methyl bedeutet,
R⁴ für Wasserstoff, geradkettiges oder verzweigtes C₁-C₃-Alkyl oder Cyclopropyl,
R^{4'} für Wasserstoff oder Methyl,
- A: für C-R7 steht, worin
R⁷ für Wasserstoff, Chlor, Fluor, Methoxy steht oder auch gemeinsam mit R¹ eine Brücke der Struktur 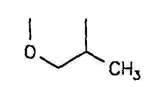
bilden kann,
und deren pharmazeutisch verwendbare Hydrate und Säureadditionsalze sowie die Alkali-, Erdalkali-, Silber- und Guanidiniumsalze der zugrundeliegenden Naphthyridoncarbonsäuren.

Weiterhin wurde gefunden, daß man Verbindungen der Formel (I) erhält, wenn man Verbindungen der Formel (II) 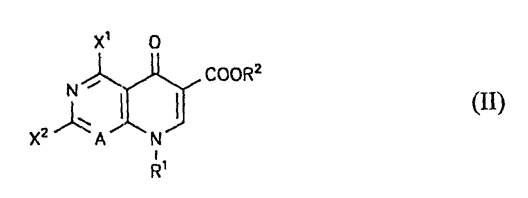 in welcher
- R¹, R², X¹, und A: die oben angegebene Bedeutung haben und
- X²: für Halogen, insbesondere Fluor oder Chlor, steht,
mit Verbindungen der Formel (III)

Z-H (III)

in welcher
- Z: die oben angegebene Bedeutung hat,
gegebenenfalls in Gegenwart von Säurefängern umsetzt.

Verwendet man beispielsweise 1-Cyclopropyl-7,8-dichlor-1,4-dihydro-4-oxo-1,6-naphthyridin-3-carbonsäure und 4-Methylamino-1,3,3a,4,7,7a-hexahydroisoindol, so kann der Reaktionsverlauf durch das folgende Formelschema wiedergegeben werden:

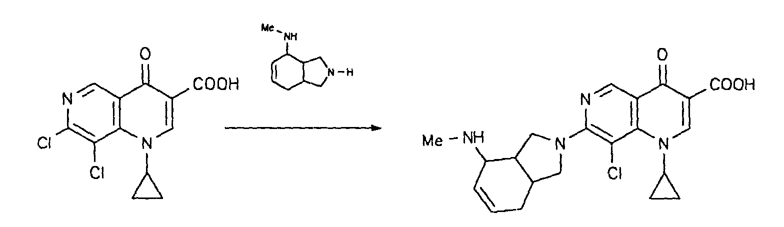

Die als Ausgangsverbindungen verwendeten Verbindungen der Formel (II) sind zum Teil bekannt und teilweise neu. Sie können gegebenenfalls als Racemate, reine Enantiomere oder Diasteromere eingesetzt werden.

Weiterhin wurde gefunden, daß man Verbindungen der Formel (II) erhält, wenn man Verbindungen der Formel (IV) 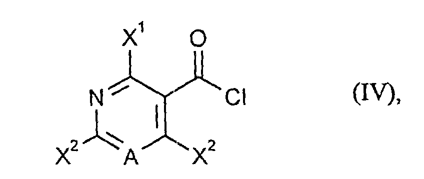 in welcher X¹, X² und A die oben angegebene Bedeutung haben in einer Reaktionsfolge, wie sie in den präparativen Beispielen Ag. bis Aj. beispielhaft beschrieben ist, umsetzt.

Weiterhin wurde gefunden, daß man Verbindungen der Formel (IV) erhält, in denen X¹ für Wasserstoff, X² für Chlor und A für CF und CCl steht, wenn man Verbindungen der Formel (V) 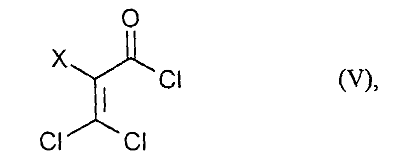 in denen X für Chlor oder Fluor steht in einer Reaktionsfolge, wie sie in den präparativen Beispielen Aa. bis Af. beispielhaft beschrieben ist, umsetzt.

Weiterhin wurde gefunden, daß man eine Verbindung der Formel (IVa) erhält, 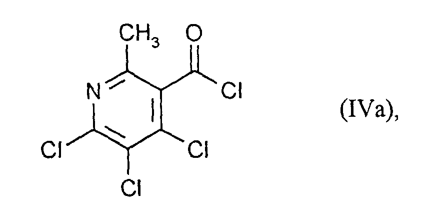 wenn man Trichloracrylsäurechlorid in einer Reaktionsfolge, wie sie in den Beispielen Ia. bis Id. beschrieben ist, umsetzt.

Die Ausgangsverbindungen für die Reaktionsfolgen Ag. bis Aj. und Ia. bis Id. sind bis auf das 3,3-Dichlor-2-fluoracrylsäurechlorid bekannt. 3,3-Dichlor-2-fluoracrylsäurechlorid läßt sich durch partielle Hydrolyse aus dem bekannten 2-Fluor-1,1,3,3,3-pentachlorpropen herstellen und ist Gegenstand der DE-A 4 401 099.

Die als Ausgangsverbindungen verwendeten Amine der Formel (III) sind in EP 520 240 und EP 588 166 beschrieben worden. Chirale Amine können sowohl als Racemate, als auch als enantiomeren- oder diastereomerereine Verbindungen eingesetzt werden.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretriamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verbindungen verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem großen Bereich variiert werden. Im allgemeinen arbeitet man zwischen etwa 0 und 200°C, vorzugsweise zwischen 20 und 140°C.

Die Umsetzung kann bei Normaldruck, aber auch bei erhöhten Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können während der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. McOmie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff, bei Temperaturen von etwa 0-100°C, vorzugsweise 0 bis 50°C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittels wie Methanol, Ethanol, Aceton oder Acetonitril. Man kann auch äquivalente Mengen Betain und Säure in Wasser oder einem Alkohol wie Glykolmonomethylether erhitzen und anschließend bis zur Trockenen eindampfen oder das ausgefallene Salz absaugen. Als pharmazeutisch verwendbare Salze sind beispielweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Verbindungen werden beispielsweise durch Lösen des Betains in unterschüssiger Alkali- oder Erdalkalilauge, Filtration von ungelösten Betain und Eindampfen des Filtrats bis zur Trockene erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Verbindungen wirken stark antibiotisch und zeigen bei geringer Toxizität ein breites antibakterielles Spektrum gegenüber grampositiven und gramnegativen Keimen, vor allem auch gegenüber solchen, die gegen verschiedene Antibiotika, wie zum Beispiel Penicilline, Cephalosporine, Aminoglykoside, Sulfonamide, Tetracycline sowie gegen handelsübliche Chinolone, resistent sind.

Diese wertvollen Eigenschaften ermöglichen ihre Verwendung als chemotherapeutische Wirkstoffe in der Medizin sowie als Stoffe zur Konservierung von anorganischen und organischen Materialien, zum Beispiel von Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch eine verstärkte Wirkung auf ruhende Keime aus. Bei ruhenden Bakterien, also Bakterien, die kein nachweisbares Wachstum zeigen, wirken die Verbindungen stark bakterizid. Dies bezieht sich nicht nur auf die einzusetzende Menge, sondern auch auf die Geschwindigkeit der Abtötung. Solche Ergebnisse konnten bei grampositiven und gramnegativen Bakterien, insbesondere bei Staphylococcus aureus, Pseudomonas aeruginosa, Enterococcus faecalis und Escherichia coli beobachtet werden.

Besonders wirksam sind die erfindungsgemäßen Verbindungen gegen typische und atypische Mykobakterien und Helicobacter pylori sowie gegen bakterienähnliche Mikroorganismen, wie zum Beispiel Mykoplasmen und Rickettsien. Sie sind daher besonders gut in der Human- und Tiermedizin zur Prophylaxe und Chemotherapie von lokalen und systemischen Infektionen geeignet, die durch diese Erreger hervorgerufen werden.

Die Verbindungen eignen sich ferner besonders gut zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die erfindungsgemäßen Verbindungen können auch mit β-Lactamderivaten wie zum Beispiel Cephalosporinen oder Penemen über kovalente Bindungen zu sogenannten Dual-Action-Derivaten verknüpft werden.

Die minimalen Hemmkonzentrationen (MHK) wurden durch ein Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt circa 10⁴ koloniebildende Partikel enthielt. Die beimpften Agarplatten wurden bei 37°C bebrütet und das Keimwachstum nach circa 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich zu 7-(3-Aminopyrrolidin-1-yl)-1-cyclopropyl-8-fluor-1,4-dihydro-4-oxo-1,6-naphthyridin-3-carbonsäure (Journal of Heterocyclic Chemistry **30** (1993), 855) als Referenzverbindung aufgeführt.

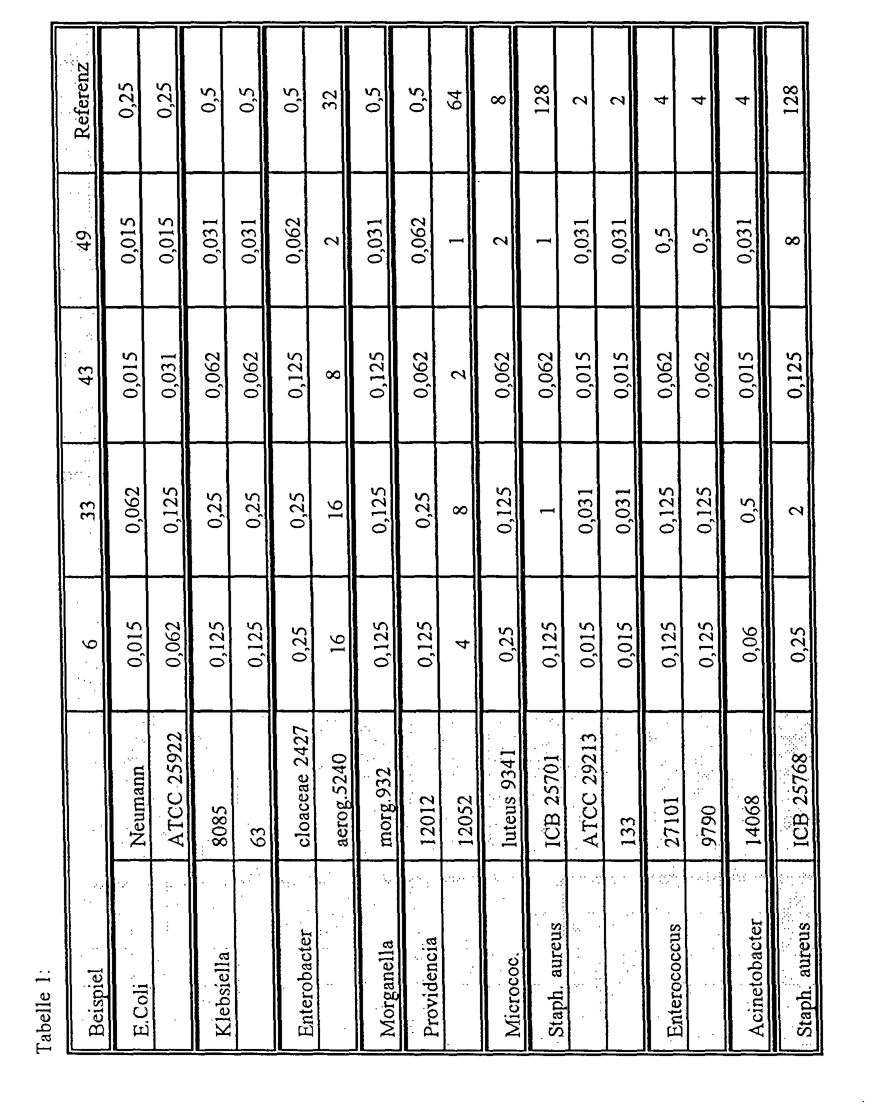

### Herstellung der Zwischenprodukte

### Beispiel A:

### 7,8-Dichlor-1-cyclopropyl-1,4-dihydro-4-oxo-1,6-naphthyridin-3-carbonsäure

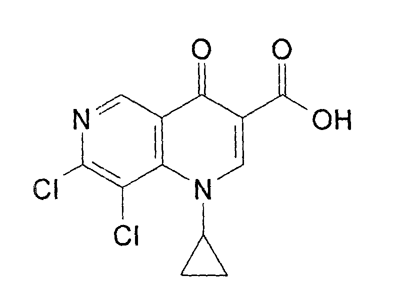

### a. 4,5,5-Trichlor-3-oxo-4-pentensäureethylester

16,9 g (0,695 Mol) Magnesium-Späne werden in 35 ml Ethanol vorgelegt und die Reaktion mit 3,5 ml Tetrachlormethan gestartet. Anschließend wird ein Gemisch aus 112 g (0,7 Mol) Malonsäurediethylester, 280 ml Toluol und 75 ml Ethanol so zugetropft, daß die Temperatur 50-60°C beträgt. Anschließend wird eine Stunde bei dieser Temperatur nachgerührt. Nach Abkühlen auf -10 bis -5 °C wird die Lösung von 135,8 g (0,7 Mol) Trichloracrylsäurechlorid in 70 ml Toluol zugetropft, eine Stunde bei 0°C und anschließend unter Erwärmung auf Raumtemperatur über Nacht nachgerührt.

Der Ansatz wird auf Eiswasser gegeben, mit 35 ml konzentrierter Schwefelsäure versetzt und die Phasen getrennt. Die wäßrige Phase wird mit Toluol extrahiert, die vereinigten organischen Phasen mit gesättigter Natriumchloridlösung gewaschen, über Natriumsulfat getrocknet und einrotiert.

Der Rückstand wird in 370 ml Wasser mit 1,1 p-Toluolsulfonsäure vier Stunden zum Rückfluß erhitzt. Es wird mit Dichlormethan extrahiert, mit gesättigter Natriumchloridlösung gewaschen, über Natriumsulfat getrocknet und das Lösungsmittel im Vakuum entfernt.
- Ausbeute:: 160 g (93% der Theorie)
- Siedepunkt:: 134-136°C (15 mbar)

### b. 4,5,5-Trichlor-2-ethoxymethylen-3-oxo-4-pentensäureethylester

305 g (1,24 Mol) 4,5,5-Trichlor-3-oxo-4-pentencarbonsäureethylester werden mit 275 g (1,86 Mol) Orthoameisensäureethylester und 316,0 g Essigsäureanhydrid drei Stunden auf 150-160°C erwärmt. Alle flüchtigen Komponenten werden zunächst im Vakuum, dann im Hochvakuum bis 100°C enfernt.
Ausbeute: 312,0 g (83% der Theorie)

### c. 2-Aminomethylen-4,5,5-trichlor-3-oxo-4-pentensäureethylester

180,6 g (0,6 Mol) 4,5,5-Trichlor-2-ethoxy-3-oxo-4-pentensäureethylester werden in 600 ml Ethanol vorgelegt und unter Eiskühlung 90 ml konzentrierte wäßrige Ammoniaklösung (25%) so zugetropft, daß die Temperatur 10°C nicht überschreitet.

Der Ansatz wird mit 1,2 1 Wasser versetzt, im Eisbad gekühlt und das auskristallisierende Produkt isoliert. Der Niederschlag wird mit Ethanol/Wasser 1:2 gewaschen.
Ausbeute: 143,1 g (87% der Theorie)
Schmelzpunkt: 105-106°C

### d. 5,6-Dichlor-4-hydroxy-nicotinsäureethylester

131 g (0,48 Mol) 2-Aminomethylen-4,5,5-trichlor-3-oxo-4-pentensäureethylester werden in 1,2-Dichlorbenzol drei Stunden auf 180°C erwärmt. Die flüchtigen Komponenten werden im Vakuum bis 90°C und anschließend im Hochvakuum bis 80°C entfernt.
Ausbeute: 116,5 g (Rohprodukt)
Schmelzpunkt: 92-94°C (aus Cyclohexan)

### e. 5,6-Dichlor-4-hydroxy-nicotinsäure

114 g (0,483 Mol) 5,6-Dichlor-4-hydroxy-nicotinsäureethylester werden in 1200 ml Wasser mit 58 g (1,03 Mol) Kaliumhydroxid zwei Stunden unter Rückfluß gekocht. Der abgekühlte Ansatz wird mit Aktivkohle gerührt, filtriert und das Filtrat unter Eiskühlung mit halbkonzentrierter Salzsäure auf pH 1 gebracht. Das ausfallende Produkt wird isoliert, mit Wasser gewaschen und getrocknet.
Ausbeute: 84,2 g (83% der Theorie)
Schmelzpunkt: 208-210°C (unter Zersetzung)

### f. 4,5,6-Trichlornicotinsäurechlorid

84 g (0,404 Mol) 5,6-Dichlor-4-hydroxynicotinsäure werden mit 380 ml Phosphoroxychlorid und einem Tropfen DMF langsam zum Rückfluß erhitzt. Nach zwei Stunden wird das Phosphoroxychlorid bei ca. 100°C im Vakuum abdestilliert. Der Rückstand wird auf Eiswasser gegeben, mit Chloroform extrahiert, über Natriumsulfat getrocknet und einrotiert.

Der Rückstand wird in 200 ml Thionylchlorid dreißig Minuten zum Rückfluß erhitzt, das Thionylchlorid im Vakuum abdestilliert und der Rückstand direkt im Hochvakuum destilliert.
Ausbeute: 78,8 g (79% der Theorie)
Siedepunkt: 96-98°C (0,14 mbar)
Schmelzpunkt: 41-42°C

### g. 2-(4,5,6-Trichlornicotinoyl)-essigsäureethylester

16,9 g (0,7 Mol) Magnesiumspäne werden in 35 ml Ethanol vorgelegt und die Reaktion mit 3,5 ml Tetrachlormethan gestartet. Anschließend wird die Mischung aus 112 g (0,7 Mol) Malonsäurediethylester, 280 ml Toluol und 75 ml Ethanol so zugetropft, daß die Temperatur zwischen 50 und 60°C liegt. Anschließend wird noch eine Stunde bei dieser Temperatur nachgerührt.

Bei -10 bis -5°C wird die Lösung von 171,5 g (0,7 Mol) 4,5,6-Trichlornicotinsäurechlorid in 70 ml Toluol zugetropft und eine Stunde bei 0°C und anschließend unter Erwärmung auf Raumtemperatur über Nacht nachgerührt.

Der Ansatz wird mit Eiswasser versetzt und 35 ml konzentrierte Schwefelsäure zugetropft. Die Phasen werden getrennt und die wäßrige Phase nochmals mit Dichlormethan extrahiert. Die vereinigten organischen Phasen werden mit gesättigter Natriumchloridlösung gewaschen, über Natriumsulfat getrocknet und einrotiert.

Der Rückstand wird mit 1,1 g para-Toluolsulfonsäure in 350 ml Wasser vier Stunden unter Rückfluß gekocht. Der abgekühlte Ansatz wird mit Dichlormethan extrahiert, die organischen Phasen über Natriumsulfat getrocknet und das Lösungsmittel im Vakuum entfernt.
Ausbeute: 116,5 g (56% der Theorie)
Schmelzpunkt: 79-80°C

### h. 2-(4,5,6-Trichlornicotinoyl)-3-ethoxy-acrylsäureethylester

87,0 g (0,29 Mol) 2-(4,5,6-Trichlornicotinoyl)-essigsäureethylester werden mit 56,2 g (0,38 Mol) Orthoameisensäureethylester und 65,2 g Essigsäureanhydrid zwei Stunden auf 150-160°C erwärmt. Alle flüchtigen Komponenten werden zunächst im Vakuum dann im Hochvakuum bis 100°C entfernt.
Ausbeute: 93 g (90% der Theorie)

### i. 2-(4,5,6-Trichlornicotinoyl)-3-cyclopropylamino-acrylsäureethylester

22,3 g (0,063 Mol) 2-(4,5,6-Trichlornicotinoyl)-3-ethoxyacrylsäureethylester werden bei 0°C in 95 ml Ethanol vorgelegt und 3,6 g (0,063 Mol) Cyclopropylamin zugetropft. Es wird zwei Stunden bei Raumtemperatur nachgerührt, mit 95 ml Wasser versetzt, auf 0°C gekühlt und das ausfallende Produkt isoliert.
Ausbeute: 17,5 g (76% der Theorie)
Schmelzpunkt: 126-128°C

### j. 7,8-Dichlor-1-cyclopropyl-1,4-dihydro-4-oxo-1,6-naphthyridin-3-carbonsäureethylester

17,5 g (0,048 Mol) 2-(4,5,6-Trichlornicotinoyl)-3-cyclopropylaminoacrylsäureethylester werden mit 7,7 g (0,056 Mol) Kaliumcarbonat in 100 ml DMF zwei Stunden auf 60°C erwärmt. Der abgekühlte Ansatz wird mit Eiswasser versetzt und das Produkt isoliert.
Ausbeute: 15 g (95% der Theorie)
Schmelzpunkt: 197-199°C

### k. 7,8-Dichlor-1-cyclopropyl-1,4-dihydro-4-oxo-1,6-naphthyridin-3-carbonsäure

14,6 g (0,045 Mol) 7,8-Dichlor-1-cyclopropyl-1,4-dihydro-4-oxo-1,6-naphthyridin-3-carbon-säureethylester werden in einer Mischung aus 59 ml Essigsäure, 59 ml Wasser und 5,9 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 12,5 g (92% der Theorie)
Schmelzpunkt: 215-217°C

### Beispiel B:

### 7,8-Dichlor-1-(2,4-difluorphenyl)-1,4-dihydro-4-oxo-1,6-naphthyridin-3-carbonsäure

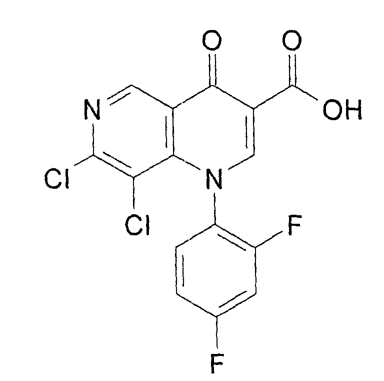

### a. 2-(4,5,6-Trichlornicotinoyl)-3-(2,4-difluorphenyl)amino-acrylsäureethylester

10,57 g (0,03 Mol) 2-(4,5,6-Trichlornicotinoyl)-3-ethoxyacrylsäureethylester werden bei 0°C in 45 ml Ethanol vorgelegt und 4,2 g (0,033 Mol) 2,4-Difluoranilin zugetropft. Es wird zwei Stunden bei Raumtemperatur nachgerührt, mit 45 ml Wasser versetzt, auf 0°C gekühlt und das ausfallende Produkt isoliert.
Ausbeute: 11,0 g (84 % der Theorie)
Schmelzpunkt: 128-130°C

### b. 7,8-Dichlor-1-(2,4-difluorphenyl)-1,4-dihydro-4-oxo-1,6-naphthyridin-3-carbonsäureethylester

10,5 g (0,024 Mol) 2-(4,5,6-Trichlornicotinoyl)-3-(2,4-difluorphenyl)aminoacrylsäureethylester werden mit 4,8 g (0,028 Mol) Kaliumcarbonat in 50 ml DMF über Nacht bei Raumtemperatur gerührt. Der Ansatz wird mit Eiswasser versetzt und das Produkt isoliert.
Ausbeute: 8,7 g (90% der Theorie)
Schmelzpunkt: 199-201°C

### c. 7,8-Dichlor-1-(2,4-difluorphenyl)-1,4-dihydro-4-oxo-1,6-naphthyridin-3-carbonsäure

8,6 g (0,022 Mol) 7,8-Dichlor-1-(2,4-difluorphenyl)-1,4-dihydro-4-oxo-1,6-naphthyridin-3-carbonsäureethylester werden in einer Mischung aus 29 ml Essigsäure, 29 ml Wasser und 2,9 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 7,2 g (88% der Theorie)
Schmelzpunkt: 206-208°C

### Beispiel C:

### 7,8-Dichlor-1-ethyl-1,4-dihydro-4-oxo-1,6-naphthyridin-3-carbonsäure

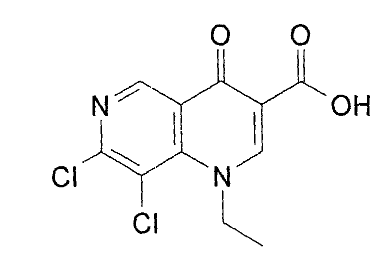

### a. 2-(4,5,6-Trichlornicotinoyl)-3-ethylamino-acrylsäureethylester

10,57 g (0,03 Mol) 2-(4,5,6-Trichlornicotinoyl)-3-ethoxyacrylsäureethylester werden bei 0°C in 45 ml Ethanol vorgelegt und 2,21 ml (0,033 Mol) Ethylaminlösung (70 %) zugetropft. Es wird zwei Stunden bei Raumtemperatur nachgerührt, mit 45 ml Wasser versetzt, auf 0°C gekühlt und das ausfallende Produkt isoliert.
Ausbeute: 7,9 g (75% der Theorie)
Schmelzpunkt: 133-135°C

### b. 7,8-Dichlor-1-ethyl-1,4-dihydro-4-oxo-1,6-naphthyridin-3-carbonsäureethylester

7,5 g (0,024 Mol) 2-(4,5,6-Trichlornicotinoyl)-3-ethylamino-acrylsäureethylester werden mit 3,37 g (0,0244 Mol) Kaliumcarbonat in 50 ml DMF über Nacht bei Raumtemperatur gerührt. Der Ansatz wird mit Eiswasser versetzt und das Produkt isoliert.
Ausbeute: 6,4 g (84% der Theorie)
Schmelzpunkt: 127-128°C

### c. 7,8-Dichlor-1-ethyl-1,4-dihydro-4-oxo-1,6-naphthyridin-3-carbonsäure

6,2 g (0,0196 Mol) 7,8-Dichlor-1-ethyl-1,4-dihydro-4-oxo-1,6-naphthyridin-3-carbonsäure-ethylester werden in einer Mischung aus 26 ml Essigsäure, 26 ml Wasser und 2,6 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 5,5 g (98% der Theorie)
Schmelzpunkt: 219-220°C

### Beispiel D:

7,8-Dichlor-1-(4-fluorphenyl)-1,4-dihydro-4-oxo-1,6-naphthyridin-3-carbonsäure

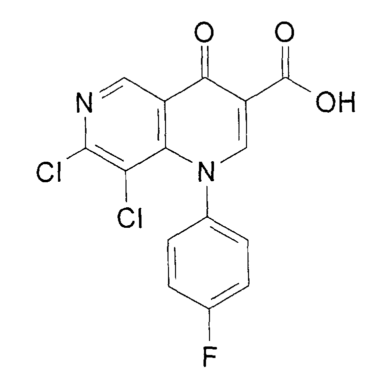

### a. 2-(4,5,6-Trichlornicotinoyl)-3-(4-fluorphenyl)amino-acrylsäureethylester

5,0 g (0,014 Mol) 2-(4,5,6-Trichlornicotinoyl)-3-ethoxyacrylsäureethylester werden bei 0°C in 21 ml Ethanol vorgelegt und 1,7 g (0,0154 Mol) 4-Fluoranilin zugetropft. Es wird zwei Stunden bei Raumtemperatur nachgerührt, mit 45 ml Wasser versetzt, auf 0°C gekühlt und das ausfallende Produkt isoliert. Das Rohprodukt wird mit Wasser/Ethanol 1:1 gewaschen.
Ausbeute: 4,9 g (83% der Theorie)
Schmelzpunkt: 126-127°C

### b. 7,8-Dichlor-1-(4-fluorphenyl)-1,4-dihydro-4-oxo-1,6-naphthyridin-3-carbonsäureethylester

4,8 g (0,012 Mol) 2-(4,5,6-Trichlornicotinoyl)-3-(4-fluorphenyl)amino-acrylsäureethylester werden mit 1,9 g (0,0138 Mol) Kaliumcarbonat in 24 ml DMF über Nacht bei Raumtemperatur gerührt. Der Ansatz wird mit Eiswasser versetzt und das Produkt isoliert. Das Rohprodukt wird mit Acetonitril verrührt.
Ausbeute: 3,36 g (78% der Theorie)
Schmelzpunkt: 219-224°C

### c. 7,8-Dichlor-1-(4-fluorphenyl)-1,4-dihydro-4-oxo-1,6-naphthyridin-3-carbonsäure

3,3 g (0,0087 Mol) 7,8-Dichlor-1-(4-fluorphenyl)-1,4-dihydro-4-oxo-1,6-naphthyridin-3-carbonsäureethylester werden in einer Mischung aus 11,5 ml Essigsäure, 11,5 ml Wasser und 1,15 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 2,9 g (94% der Theorie)
Schmelzpunkt: 274-276°C

### Beispiel E:

### 7,8-Dichlor-1-(2-fluorethyl)-1,4-dihydro-4-oxo-1,6-naphthyridin-3-carbonsäure

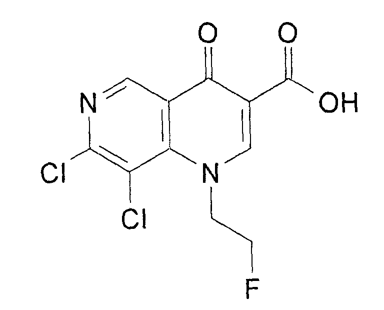

### a. 2-(4,5,6-Trichlornicotinoyl)-3-(2-fluorethyl)amino-acrylsäureethylester

5,0 g (0,0142 Mol) 2-(4,5,6-Trichlornicotinoyl)-3-ethoxyacrylsäureethylester und 1,4 g (0,0142 Mol) Fluorethylaminhydrochlorid werden in einer Mischung aus 22 ml Dichlormethan und 8,5 ml Wasser bei 0°C vorgelegt und die Lösung von 1,2 g Natriumhydrogencarbonat in 14 ml Wasser zugetropft. Es wird drei Stunden bei Raumtemperatur nachgerührt, die Phasen werden getrennt, die organische Phase über Natriumsulfat getrocknet und einrotiert.
Ausbeute: 5,7 g Rohprodukt

### b. 7,8-Dichlor-1-(2-fluorethyl)-1,4-dihydro-4-oxo-1,6-naphthyridin-3-carbonsäure-ethylester

5,5 g (0,0148 Mol) 2-(4,5,6-Trichlornicotinoyl)-3-(2-fluorethyl)amino-acrylsäureethylester werden mit 2,4 g (0,0174 Mol) Kaliumcarbonat in 30 ml DMF über Nacht bei Raumtemperatur gerührt. Der Ansatz wird mit Eiswasser versetzt und das Produkt isoliert.
Ausbeute: 3,5 g (71% der Theorie)
Schmelzpunkt: 168-169°C

### c. 7,8-Dichlor-1-(2-fluorethyl)-1,4-dihydro-4-oxo-1,6-naphthyridin-3-carbonsäure

3,33 g (0,01 Mol) 7,8-Dichlor-1-(2-fluorethyl)-1,4-dihydro-4-oxo-1,6-naphthyridin-3-carbonsäureethylester werden in einer Mischung aus 13 ml Essigsäure, 13 ml Wasser und 1,3 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 2,7 g (88% der Theorie)
Schmelzpunkt: 226-228°C

### Beispiel F:

### 7,8-Dichlor-1-[(1R,2S)-2-fluorcyclopropyl]-1,4-dihydro-4-oxo-1,6-naphthyridin-3-carbonsäure

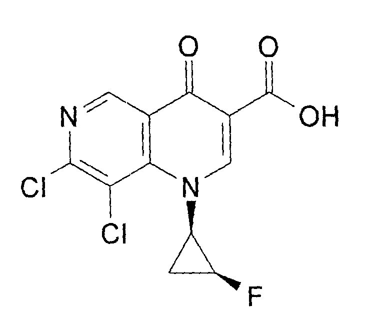

### a. 2-(4,5,6-Trichlornicotinoyl)-3-[(1R,2S)-2-fluorcyclopropyl]aminoacrylsäureethylester

6,35 g (0,018 Mol) 2-(4,5,6-Trichlornicotinoyl)-3-ethoxyacrylsäureethylester und 2,0 g (0,018 Mol) (1R,2S)-2-Fluorcyclopropylaminhydrochlorid werden in einer Mischung aus 27 ml Dichlormethan und 10,8 ml Wasser bei 0°C vorgelegt und die Lösung von 1,5 g Natriumhydrogencarbonat in 18 ml Wasser zugetropft. Es wird vier Stunden bei Raumtemperatur nachgerührt, die Phasen werden getrennt, die organische Phase über Natriumsulfat getrocknet und einrotiert.
Ausbeute: 6,6 g Rohprodukt

### b. 7,8-Dichlor-1-[(1R,2S)-2-fluorcyclopropyl]-1,4-dihydro-4-oxo-1,6-naphthyridin-3-carbonsäureethylester

6,6 g (0,017 Mol) 2-(4,5,6-Trichlornicotinoyl)-3-[(1R,2S)-2-fluorcyclopropyl]-amino-acrylsäureethylester werden mit 2,7 g (0,019 Mol) Kaliumcarbonat in 35 ml DMF über Nacht bei Raumtemperatur gerührt. Der Ansatz wird mit Eiswasser versetzt und das Produkt isoliert.
Ausbeute: 4,6 g (78% der Theorie)
Schmelzpunkt: 198-200°C

### c. 7,8-Dichlor-1-[(1R,2S)-2-fluorcyclopropyl]-1,4-dihydro-4-oxo-1,6-naphthyridin-3-carbonsäure

4,4 g (0,0128 Mol) 7,8-Dichlor-1-[(1R,2S)-2-fluorcyclopropyl]-1,4-dihydro-4-oxo-1,6-naphthyridin-3-carbonsäureethylester werden in einer Mischung aus 14,6 ml Essigsäure, 14,6 ml Wasser und 1,5 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 3,6 g (88% der Theorie)
Schmelzpunkt: 204-206°C

### Beispiel G:

### 9-Chlor-3-methyl-6-oxo-3,6-dihydro-2H-1-oxa-3a,8-diazaphenalen-5-carbonsäure

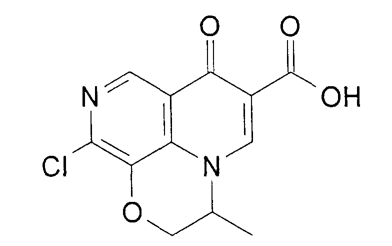

### a. 2-(4,5,6-Trichlornicotinoyl)-3-(1-hydroxy-2-propyl)aminoacrylsäureethylester

5,0 g (0,014 Mol) 2-(4,5,6-Trichlornicotinoyl)-3-ethoxyacrylsäureethylester werden bei 0°C in 21 ml Ethanol vorgelegt und 1,16 g (0,0154 Mol) 2-Aminopropanol zugetropft. Es wird zwei Stunden bei Raumtemperatur nachgerührt, mit 45 ml Wasser versetzt, mit Dichlormethan extrahiert, über Natriumsulfat getrocknet und einrotiert.
Ausbeute: 5,3 g Rohprodukt

### b. 7,8-Dichlor-1-(1-hydroxy-2-propyl)-1,4-dihydro-4-oxo-1,6-naphthyridin-3-carbonsäureethylester

5,2 g (0,014 Mol) 2-(4,5,6-Trichlornicotinoyl)-3-(1-hydroxy-2-propyl)aminoacrylsäureethylester werden mit 2,3 g (0,0167 Mol) Kaliumcarbonat in 28 ml DMF über Nacht bei Raumtemperatur gerührt. Der Ansatz wird mit Eiswasser versetzt und das Produkt isoliert. Die Mutterlauge wird mit Chloroform extrahiert und die organische Phase einrotiert. Die Rohprodukte werden mit Acetonitril verrührt.
Ausbeute: 2,42 g (51% der Theorie)
Schmelzpunkt: 189-192°C

### c. 9-Chlor-3-methyl-6-oxo-3,6-dihydro-2H-1-oxa-3a,8-diazaphenalen-5-carbonsäure-ethylester

2,42 g (0,007 Mol) 7,8-Dichlor-1-(1-hydroxy-2-propyl)-1,4-dihydro-4-oxo-1,6-naphthyridin-3-carbonsäureethylester werden mit 1,15 g (0,0083 Mol) Kaliumcarbonat in 14 ml Dimethylformamid eine Stunde auf 80°C erwärmt. Nach Zugabe von Eiswasser wird das ausgefallene Produkt isoliert. Das Rohprodukt wird durch Säulenchromatographie (Laufmittel: Dichlormethan/Methanol 98:2) gereinigt.
Ausbeute: 1,0 g (46 % der Theorie)
Schmelzpunkt: 185-186°C

### d. 9-Chlor-3-methyl-6-oxo-3,6-dihydro-2H-1-oxa-3a,8-diazaphenalen-5-carbonsäure

0,95 g (3,1 mMol) 9-Chlor-3-methyl-6-oxo-3,6-dihydro-2H-1-oxa-3a,8-diazaphenalen-5-carbonsäureethylester werden in einer Mischung aus 4 ml Essigsäure, 4 ml Wasser und 0,4 ml konzentrierter Schwefelsäure vier Stunden zum Rückfluß erhitzt. Der abgekühlte Ansatz wird auf Eiswasser gegeben, das Produkt isoliert und mit Wasser gewaschen.
Ausbeute: 0,65 g (94% der Theorie)
Schmelzpunkt: 218-220°C (unter Zersetzung)

### Beispiel H:

### 7-Chlor-8-fluor-1-(2,4-difluorphenyl)-1,4-dihydro-4-oxo-1,6-naphthyridin-3-carbonsäure

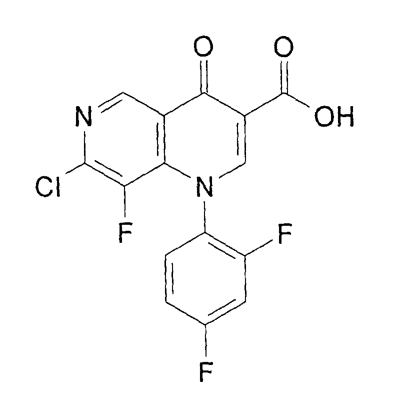

### a. 2-(4,6-Dichlor-5-fluornicotinoyl)-3-(2,4-difluorphenyl)aminoacrylsäureethylester

6,72 g (0,02 mol) 2-(4,6-Dichlor-5-fluornicotinoyl)-3-ethoxyacrylsäureethylester werden bei 0°C in 30 ml Ethanol vorgelegt und eine Lösung von 2,58 g (0,02 Mol) 2,4-Difluoranilin in 10 ml Ethanol zugetropft. Es wird dreißig Minuten bei Raumtemperatur nachgerührt, auf 0°C gekühlt und das ausfallende Produkt isoliert. Das Rohprodukt wird mit Ethanol gewaschen.
Ausbeute: 4,5 g (55 % der Theorie)
Schmelzpunkt: 125°C

### b. 7-Chlor-8-fluor-1-(2,4-difluorphenyl)-1,4-dihydro-4-oxo-1,6-naphthyridin-3-carbonsäureethylester

4,26 g (10 mMol) 2-(4,6-Dichlor-5-fluornicotinoyl)-3-(2,4-difluorphenyl)aminoacrylsäureethylester werden mit 1,65 g (12 mMol) Kaliumcarbonat in 28 ml DMF vier Stunden bei 100°C gerührt. Der Ansatz wird mit Eiswasser versetzt und das Produkt isoliert.
Ausbeute: 3,55 g (93% der Theorie)
Schmelzpunkt: 186°C

### c. 7-Chlor-8-fluor-1-(2,4-difluorphenyl)-1,4-dihydro-4-oxo-1,6-naphthyridin-3-carbonsäure

2,9 g (7,6 mMol) 7-Chlor-8-fluor-1-(2,4-difluorphenyl)-1,4-dihydro-4-oxo-1,6-naphthyridin-3-carbonsäureethylester werden in einer Mischung aus 10 ml Essigsäure, 10 ml Wasser und 1 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 2,4 g (92% der Theorie)
Schmelzpunkt: 172-174°C (unter Zersetzung)

### Beispiel I:

### 6,7-Dichlor-1-cyclopropyl-1,4-dihydro-5-methyl-4-oxo-1,6-naphthyridin-3-carbonsäure

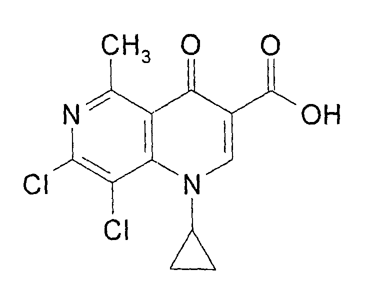

### a. 4,5,5-Trichlor-3-hydroxy-2-(1-imino-ethyl)-penta-2,4-diensäureethylester

In 1068 ml Toluol werden 383,6 g (1,98 Mol) Trichloracrylsäurechlorid gelöst und bei 10°C 255,5 g (1,98 Mol) Aminocrotonsäureethylester in 156,7 g (1,98 Mol) Pyridin zugetropft. Nach Rühren über Nacht bei Raumtemperatur wird der Ansatz 2x mit 600 ml Wasser gewaschen, die organische Phase über Natriumsulfat getrocknet und im Hochvakuum eingeengt. Das Rohprodukt wird in n-Hexan aufgeschlämmt und getrocknet.
Ausbeute: 406 g (70% der Theorie)
Schmelzpunkt: 80°C

### b. 5,6-Dichlor-4-hydroxy-2-methyl-nicotinsäureethylester

102,7 g (0,35 Mol) 4,5,5-Trichlor-3-hydroxy-2-(1-imino-ethyl)-penta-2,4-diensäureethylester werden in 1,2-Dichlorbenzol drei Stunden auf 180°C erwärmt. Die flüchtigen Komponenten werden im Hochvakuum bis 100°C entfernt. Der Rückstand wird mit Dichlormethan an Kieselgel gereinigt.
Ausbeute: 71,8 g (65 % der Theorie)

### c. 5,6-Dichlor-4-hydroxy-2-methyl-nicotinsäure

36 g (0,14 Mol) 5,6-Dichlor-4-hydroxy-2-methyl-nicotinsäureethylester werden in 88 ml Ethanol und 175 ml Wasser mit 24,2 g (0,43 Mol) Kaliumhydroxid dreißig Minuten unter Rückfluß gekocht. Der abgekühlte Ansatz wird unter Eiskühlung mit halbkonzentrierter Salzsäure auf pH 1 gebracht. Das ausfallende Produkt wird isoliert, mit Wasser gewaschen und getrocknet.
Ausbeute: 31,5 g (98% der Theorie)
Schmelzpunkt: 240°C

### d. 4,5,6-Trichlor-2-methyl-nicotinsäurechlorid

30,4 g (0,14 Mol) 5,6-Dichlor-4-hydroxy-2-methylnicotinsäure werden mit 268 ml Phosphoroxychlorid und einem Tropfen DMF langsam zum Rückfluß erhitzt. Nach zwei Stunden wird das Phosphoroxychlorid bei ca. 100°C im Vakuum abdestilliert. Der Rückstand wird auf Eiswasser gegeben, mit Dichlormethan extrahiert, über Natriumsulfat getrocknet und einrotiert.

Der Rückstand wird in 165 ml Thionylchlorid dreißig Minuten zum Rückfluß erhitzt, das Thionylchlorid im Vakuum abdestilliert und der Rückstand direkt im Hochvakuum destilliert.
Ausbeute: 22,9 g (65% der Theorie)
Siedepunkt: 103°C (0,6 mbar)

### e. 2-(4,5,6-Trichlor-2-methylnicotinoyl)-essigsäureethylester

In 272 ml absoluten Acetonitril werden 31,55 g (0,18 Mol) Kaliummonoethylmalonat, 19,29 g (0,19 Mol) Triethylamin und 21,56 g (0,23 Mol) Magnesiumchlorid vorgelegt und zwei Stunden gerührt. Anschließend werden bei Raumtemperatur 22,9 g (0,09 Mol) 4,5,6-Trichlor-2-methyl-nicotinsäurechlorid und 2,3 g (0,02 Mol) Triethylamin zugetropft und über Nacht nachgerührt. Der Ansatz wird im Vakuum eingeengt, der Rückstand in 135 ml Toluol aufgenommen und nochmals einrotiert.

Nach erneutem Aufnehmen in 135 ml Toluol werden unter Eiskühlung 130 ml 13%ige Salzsäure zugetropft, 30 Minuten nachgerührt und die Phasen getrennt. Die wäßrige Phase wird mit Toluol extrahiert, die vereinigten organischen Phasen mit Wasser gewaschen, über Natriumsulfat getrocknet und eingeengt.
Ausbeute: 29,2 g (100% der Theorie)

### f. 2-(4,5,6-Trichlor-2-methylnicotinoyl)-3-ethoxy-acrylsäureethylester

29,2 g (0,09 Mol) 2-(4,5,6-Trichlor-2-methyl-nicotinoyl)-essigsäureethylester werden mit 20,9 g (0,141 Mol) Orthoameisensäureethylester und 23,9 g (0,23 Mol) Essigsäureanhydrid zwei Stunden auf 150-160°C erwärmt. Alle flüchtigen Komponenten werden zunächst im Vakuum dann im Hochvakuum bis 80°C entfernt.
Ausbeute: 27 g (78% der Theorie)

### g. 2-(4,5,6-Trichlor-2-methyl-nicotinoyl)-3-cyclopropylamino-acrylsäureethylester

27 g (0,07 Mol) 2-(4,5,6-Trichlor-2-methyl-nicotinoyl)-3-ethoxyacrylsäureethylester werden bei 0°C in 93 ml Ethanol vorgelegt und 4,3 g (0,07 Mol) Cyclopropylamin in 34 ml Ethanol zugetropft. Es wird dreißig Minuten bei Raumtemperatur nachgerührt, auf 0°C gekühlt und das ausfallende Produkt isoliert und mit kaltem Ethanol gewaschen.
Ausbeute: 14,3 g (52% der Theorie)
Schmelzpunkt: 95°C

### h. 7,8-Dichlor-1-cyclopropyl-1,4-dihydro-5-methyl-4-oxo-1,6-naphthyridin-3-carbonsäure-ethylester

4,8 g (0,01 Mol) 2-(4,5,6-Trichlor-2-methyl-nicotinoyl)-3-cyclopropylamino-acrylsäureethylester werden mit 2,2 g (0,016 Mol) Kaliumcarbonat in 40 ml DMF vier Stunden auf 100°C erwärmt. Der abgekühlte Ansatz wird mit Eiswasser versetzt, das Produkt isoliert, mit Eiswasser gewaschen und getrocknet.
Ausbeute: 3 g (70% der Theorie)
Schmelzpunkt: 172°C

### i. 7,8-Dichlor-1-cyclopropyl-1,4-dihydro-5-methyl-4-oxo-1,6-naphthyridin-3-carbonsäure

6,2 g (0,018 Mol) 7,8-Dichlor-1-cyclopropyl-1,4-dihydro-5-methyl-4-oxo-1,6-naphthyridin-3-carbonsäureethylester werden in einer Mischung aus 62 ml Essigsäure, 43 ml Wasser und 7,4 ml konzentrierter Schwefelsäure zwei Stunden zum Rückfluß erhitzt. Der abgekühlte Ansatz wird auf Eiswasser gegeben, das Produkt isoliert und mit Wasser gewaschen.
Ausbeute: 4,3 g (76% der Theorie)
Schmelzpunkt: 212°C

### Beispiel J:

### 7,8-Dichlor-1-[(1R,2S)-2-fluorcyclopropyl]-1,4-dihydro-5-methyl-4-oxo-1,6-naphthyridin-3-carbonsäure

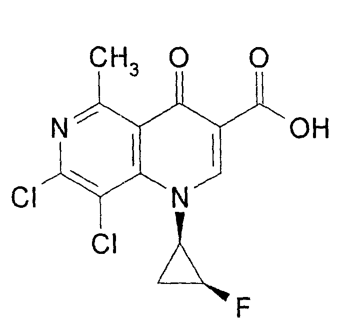

### a. 2-(4,5,6-Trichlor-2-methyl-nicotinoyl)-3-[(1R,2S)-2-fluorcyclopropyl]aminoacryl-säureethylester

6,6 g (0,018 Mol) 2-(4,5,6-Trichlor-2-methyl-nicotinoyl)-3-ethoxyacrylsäureethyl-ester und 2,0 g (0,018 Mol) (1R,2S)-2-Fluorcyclopropylaminhydrochlorid werden in einer Mischung aus 27 ml Dichlormethan und 10,8 ml Wasser bei 0°C vorgelegt und die Lösung von 1,5 g Natriumhydrogencarbonat in 18 ml Wasser zugetropft. Es wird vier Stunden bei Raumtemperatur nachgerührt, die Phasen werden getrennt, die organische Phase über Natriumsulfat getrocknet und einrotiert.
Ausbeute: 7,0 g Rohprodukt

### b. 7,8-Dichlor-1-[(1R,2S)-2-fluorcyclopropyl]-1,4-dihydro-5-methyl-4-oxo-1,6-naphthyridin-3-carbonsäureethylester

7,0 g (0,017 Mol) 2-(4,5,6-Trichlor-2-methyl-nicotinoyl)-3-[(1R,2S)-2-fluorcyclopropyl]amino-acrylsäureethylester werden mit 2,8 g (0,020 Mol) Kaliumcarbonat in 35 ml DMF über Nacht bei Raumtemperatur gerührt. Der Ansatz wird mit Eiswasser versetzt und das Produkt isoliert.
Ausbeute: 5,1 g (81% der Theorie)
Schmelzpunkt: 154-155°C

### c. 7,8-Dichlor-1-[(1R,2S)-2-fluorcyclopropyl]-1,4-dihydro-5-methyl-4-oxo-1,6-naphthyridin-3-carbonsäure

4,9 g (0,0136 Mol) 7,8-Dichlor-1-[(1R,2S)-2-fluorcyclopropyl]-1,4-dihydro-5-methyl-4-oxo-1,6-naphthyridin-3-carbonsäureethylester werden in einer Mischung aus 15,5 ml Essigsäure, 15,5 ml Wasser und 1,55 ml konzentrierter Schwefelsäure zwei Stunden zum Rückfluß erhitzt. Der abgekühlte Ansatz wird auf Eiswasser gegeben, das Produkt isoliert, mit Wasser gewaschen und das Rohprodukt mit iso-Propanol verrührt.
Ausbeute: 2,6 g (57% der Theorie)
Schmelzpunkt: 206-207°C

### Synthese der Wirkstoffe

### Beispiel 1:

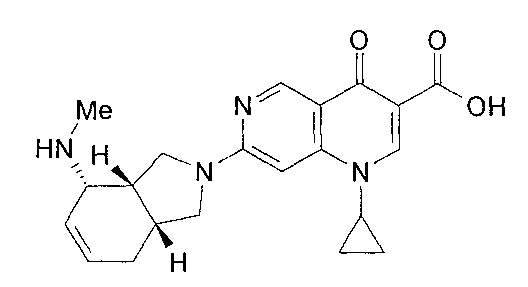

### 1-Cyclopropyl-1,4-dihydro-7-[(3aα,4β,7aα)-4-methylamino-1,3,3a,4,7,7a-hexahydroisoindol-2-yl]-4-oxo-1,6-naphthyridin-3-carbonsäure

264,5 mg (1mMol) 7-Chlor-1-cyclopropyl-1,4-dihydro-4-oxo-1,6-naphthyridin-3-carbonsäure werden mit 334,5 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril sechs Stunden zum Rückfluß erhitzt. Der Niederschlag wird isoliert und mit Acetonitril gewaschen.
Ausbeute: 330 mg (86% der Theorie)
Schmelzpunkt: 239-240°C (unter Zersetzung)

### Beispiel 2:

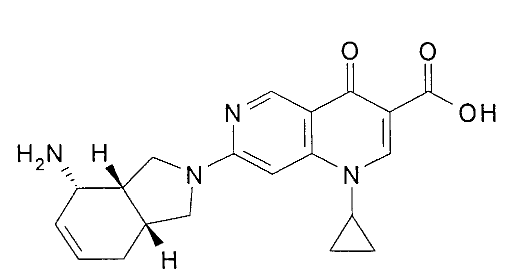

### 7-[(3aα,4β,7aα)4-Amino-1,3,3a,4,7,7a-hexahydroisoindol-2-yl]-1-cyclopropyl-1,4-dihydro-4-oxo-1,6-naphthyridin-3-carbonsäure

264,5 mg (1mMol) 7-Chlor-1-cyclopropyl-1,4-dihydro-4-oxo-1,6-naphthyridin-3-carbonsäure werden mit 303 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff acht Stunden zum Rückfluß erhitzt. Der Niederschlag wird isoliert und mit Acetonitril gewaschen.
Ausbeute: 340 mg (92% der Theorie)
Schmelzpunkt: 204-206°C (unter Zersetzung)

### Beispiel 3:

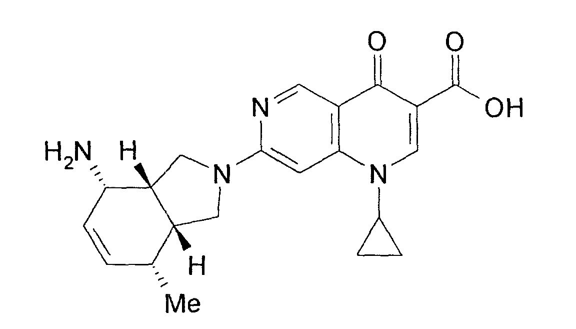

### 7-[(3aα,4β,7β,7aα)-4-Amino-7-methyl-1,3,3a,4,7,7a-hexahydroisoindol-2-yl]-1-cyclopropyl-1,4-dihydro-4-oxo-1,6-naphthyridin-3-carbonsäure

264,5 mg (1mMol) 7-Chlor-1-cyclopropyl-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff acht Stunden zum Rückfluß erhitzt. Der Niederschlag wird isoliert und mit Acetonitril gewaschen.
Ausbeute: 296 mg (77% der Theorie)
Schmelzpunkt: 204-206°C (unter Zersetzung)

### Beispiel 4:

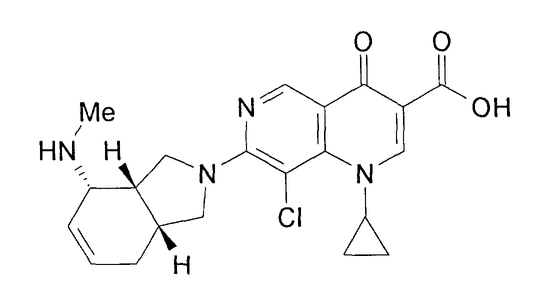

### 8-Chlor-1-cyclopropyl-1,4-dihydro-7-[(3aα,4β,7aα)-4-methylamino-1,3,3a,4,7,7a-hexahydroisoindol-2-yl]-4-oxo-1,6-naphthyridin-3-carbonsäure

299 mg (1mMol) 7,8-Dichlor-1-cyclopropyl-1,4-dihydro-4-oxo-1,6-naphthyridin-3-carbonsäure werden mit 334,5 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril vier Stunden unter Stickstoff zum Rückfluß erhitzt. Der Niederschlag wird isoliert und mit Acetonitril gewaschen.
Ausbeute: 380 mg (91% der Theorie)
Schmelzpunkt: 210-212°C (unter Zersetzung)

### Beispiel 5:

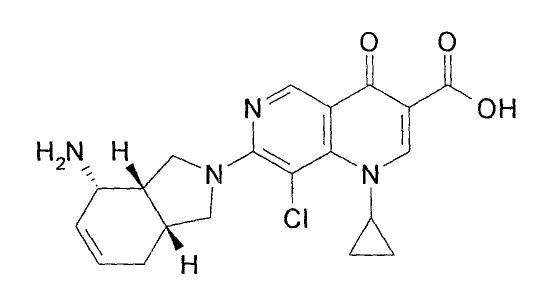

### 7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäure

1,05 g (3,5 mMol) 7,8-Dichlor-1-cyclopropyl-1,4-dihydro-4-oxo-1,6-naphthyridin-3-carbonsäure werden mit 1,063 g (7,7 mMol) (3aα,4β,7aα)-4-Amino-1,3,3a,4,7,7a-hexahydroisoindol in einem Gemisch aus 11 ml Dimethylformamid und 11 ml Acetonitril unter Stickstoff acht Stunden zum Rückfluß erhitzt. Der Niederschlag wird isoliert und mit Wasser verrührt.
Ausbeute: 1,35 g (96% der Theorie)
Schmelzpunkt: 193-195°C (unter Zersetzung)

### Beispiel 6:

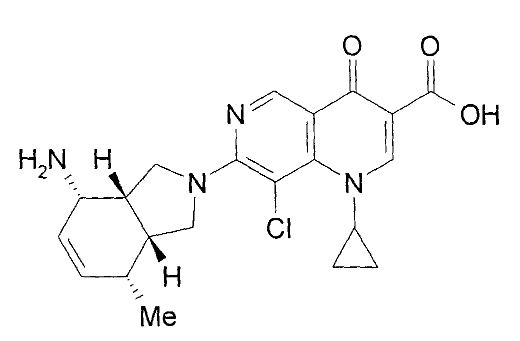

### 7-[(3aα,4β,7β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carbonsäure

1,05 g (3,5 mMol) 7,8-Dichlor-1-cyclopropyl-1,4-dihydro-4-oxo-1,6-naphthyridin-3-carbonsäure werden mit 1,17 g (7,7 mMol) (3aα,4β,7β,7aα)-4-Amino-7-methyl-1,3,3a,4,7,7a-hexahydroisoindol in einem Gemisch aus 11 ml Dimethylformamid und 11 ml Acetonitril unter Stickstoff sechs Stunden zum Rückfluß erhitzt. Der Niederschlag wird isoliert und mit Wasser verrührt.
Ausbeute: 1,2 g (82% der Theorie)
Schmelzpunkt: 210-212°C (unter Zersetzung)

### Beispiel 7:

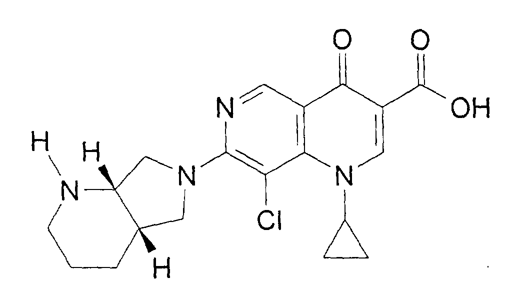

### 8-Chlor-1-cyclopropyl-1,4-dihydro-7-[(1S,6S)-2,8-diazabicyclo[4.3.0]nonan-8-yl]-4-oxo-1,6-naphthyridin-3-carbonsäure

200 mg (0,67 mMol) 7,8-Dichlor-1-cyclopropyl-1,4-dihydro-4-oxo-1,6-naphthyridin-3-carbonsäure werden mit 190 mg (1,5 mMol) (1S,6S)-2,8-Diazabicyclo[4.3.0]nonan in einem Gemisch aus 1,7 ml Dimethylformamid und 1,7 ml Acetonitril über Nacht unter Argon gerührt. Der Niederschlag wird isoliert und mit Acetonitril gewaschen. Das Rohprodukt wird an Kieselgel (Laufmittel: Dichlormethan/Methanol/wäßr. Ammoniaklösung 75:20:25) gereinigt.
Ausbeute: 56 mg (21% der Theorie)

### Beispiel 8:

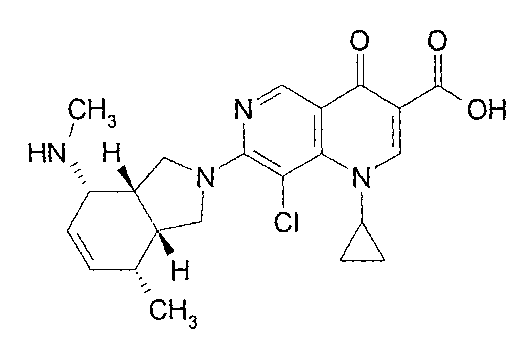

### 8-Chlor-1-cyclopropyl-1,4-dihydro-7-[(3aα,4β,7β,7aα)-7-methyl-4-methylamino-1,3,3a,4,7,7a-hexahydroisoindol-2-yl]-4-oxo-1,6-naphthyridin-3-carbonsäure

299 mg (1 mMol) 7,8-Dichlor-1-cyclopropyl-1,4-dihydro-4-oxo-1,6-naphthyridin-3-carbonsäure werden mit 360 mg (2,2 mMol) (3aα,4β,7β,7aα)-7-Methyl-4-methylamino-1,3,3a,4,7,7a-hexahydroisoindol in einem Gemisch aus 3 ml Dimethylformamid und 3 ml Acetonitril neun Stunden unter Stickstoff zum Rückfluß erhitzt. Der Niederschlag wird isoliert und mit Acetonitril gewaschen. Das Rohprodukt wird gründlich mit Wasser verrührt.
Ausbeute: 330 mg (76% der Theorie)
Schmelzpunkt: 196-198°C (unter Zersetzung)

### Beispiel 9:

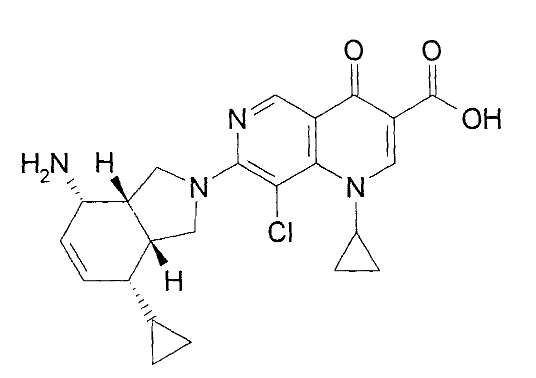

### 7-[(3aα,4β,7β,7aα)-4-Amino-7-cyclopropyl-1,3,3a,4,7,7a-hexahydroisoindol-2-yl]-8-chlor-1-cyclopropyl-1,4-dihydro-4-oxo-1,6-naphthyridin-3-carbonsäure

200 mg (0,67 mMol) 7,8-Dichlor-1-cyclopropyl-1,4-dihydro-4-oxo-1,6-naphthyridin-3-carbonsäure werden mit 260 mg (1,5 mMol) (3aα,4β,7β,7aα)-4-Amino-7-cyclopropyl-1,3,3a,4,7,7a-hexahydroisoindol in einem Gemisch aus 1,7 ml Dimethylformamid und 1,7 ml Acetonitril unter Argon neun Stunden zum Rückfluß erhitzt. Der Niederschlag wird isoliert, mit Acetonitril gewaschen und mit Wasser verrührt.
Ausbeute: 125 mg (43% der Theorie)
Schmelzpunkt: 229-230°C (unter Zersetzung)

### Beispiel 10:

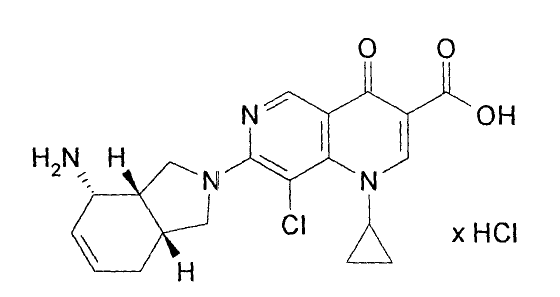

### 7-[(3aα,4β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carbonsäurehydrochlorid

6,1 g (0,015 Mol) 7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäure werden in 180 ml halbkonzentrierter Salzsäure bis zur vollständigen Lösung erwärmt. Die überschüssige Salzsäure wird im Vakuum entfernt und der Rückstand mit Acetonitril verrührt.
Ausbeute: 6,3 g (96% der Theorie)
Schmelzpunkt: 238-240°C (unter Zersetzung)

### Beispiel 11:

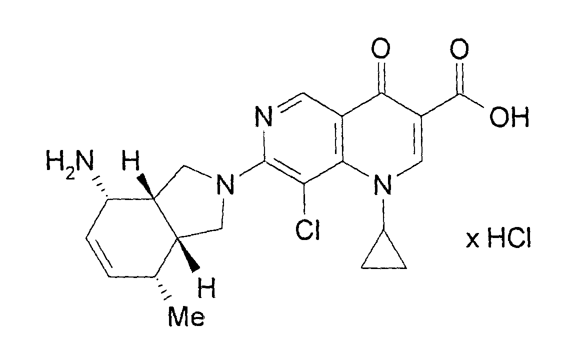

### 7-[(3aα,4β,7β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäurehydrochlorid

Analog zum Beispiel 10 wird bei der Umsetzung von 7-[(3aα,4β,7β,7aα)-4-Amino-7-methyl-1,3,3a,4,7,7a-hexahydroisoindol-2-yl)-8-chlor-1-cyclopropyl-1,4-dihydro-4-oxo-1,6-naphthyridin-3-carbonsäure die Titelverbindung erhalten.
Schmelzpunkt: 190-192°C (unter Zersetzung)

### Beispiel 12:

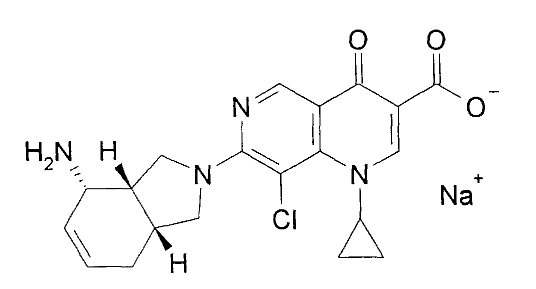

### Natrium-7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3-carboxylat

240 mg (0,6 mMol) 7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäure werden in 30 ml Wasser und 0,6 ml (0,6 mMol) 1 N Natronlauge bis zur vollständigen Lösung erwärmt. Das überschüssige Wasser wird im Vakuum entfernt und der Rückstand mit Ethanol verrührt.
Ausbeute: 210 mg (84% der Theorie)
Schmelzpunkt: >300°C

### Beispiel 13:

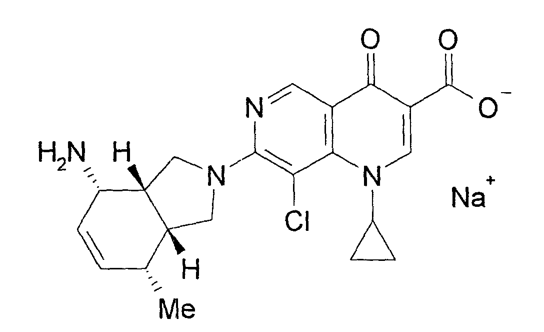

### Natrium-[7-[(3aα,4β,7β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carboxylat]

Analog zum Beispiel 12 wird bei der Umsetzung von 7-[(3aα,4β,7β,7aα)-4-Amino-7-methyl-1,3,3a,4,7,7a-hexahydroisoindol-2-yl)-8-chlor-1-cyclopropyl-1,4-dihydro-4-oxo-1,6-naphthyridin-3-carbonsäure die Titelverbindung erhalten.
Schmelzpunkt: 230-232°C (unter Zersetzung)

### Beispiel 14:

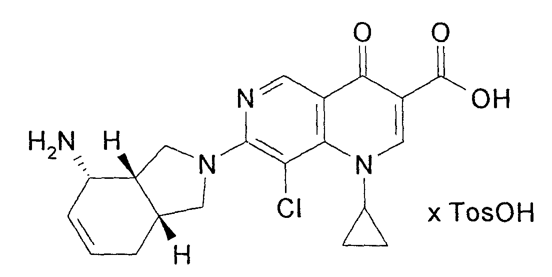

### 7-[(3aα,4β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carbonsäuretosylat

240 mg (0,6 mMol) 7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3 -carbonsäure werden mit 114 mg (0,6 mMol) p-Toluolsulfonsäure in 30 ml Wasser bis zur vollständigen Lösung erwärmt. Das Wasser wird im Vakuum entfernt und das Produkt getrocknet.
Ausbeute: 240 mg (70% der Theorie)
Schmelzpunkt: 296-298°C (unter Zersetzung)

### Beispiel 15:

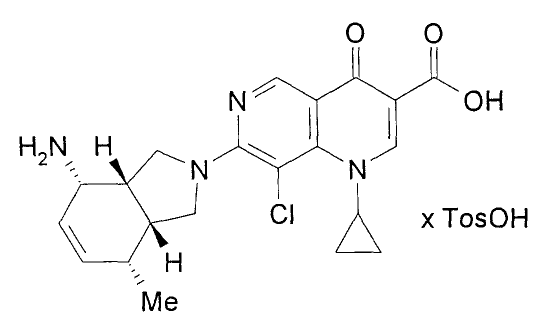

### 7-[(3aα,4β,7β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäuretosylat

Analog zum Beispiel 14 wird bei der Umsetzung von 7-[(3aα,4β,7β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carbonsäure die Titelverbindung erhalten.
Schmelzpunkt: 288-290°C (unter Zersetzung)

### Beispiel 16:

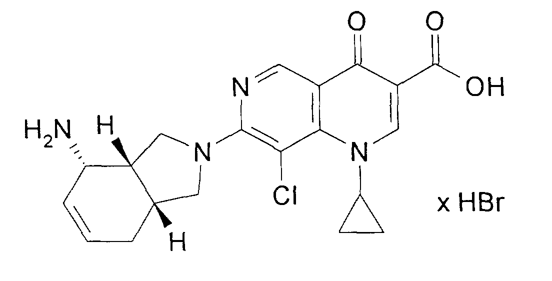

### 7-[(3aα,4β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carbonsäurehydrobromid

240 mg (0,6 mMol) 7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäure werden mit 100 mg (0,6 mMol) 48%iger Bromwasserstoffsäure in 30 ml Wasser bis zur vollständigen Lösung erwärmt. Das Wasser wird im Vakuum entfernt und das Produkt mit Ethanol verrührt.
Ausbeute: 270 mg (70% der Theorie)
Schmelzpunkt: 236-238°C (unter Zersetzung)

### Beispiel 17:

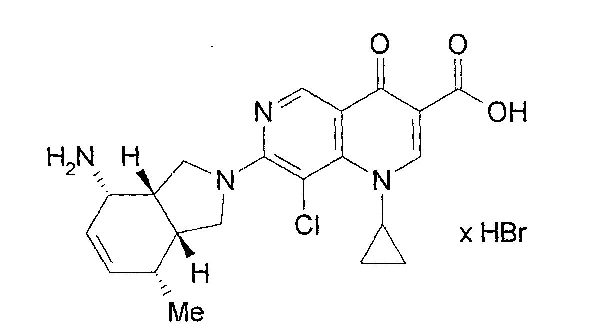

### 7-[(3aα,4β,7β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäurehydrobromid

Analog zum Beispiel 16 wird bei der Umsetzung von 7-[(3aα,4β,7β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carbonsäure die Titelverbindung erhalten.
Schmelzpunkt: 228-230°C (unter Zersetzung)

### Beispiel 18:

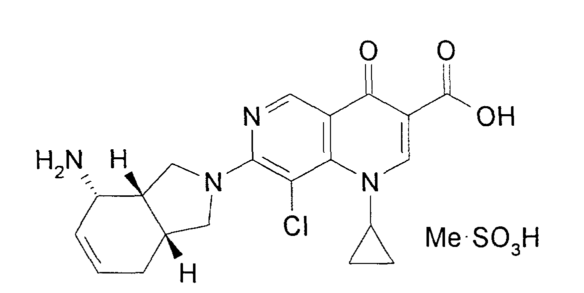

### 7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäuremesylat

240 mg (0,6 mMol) 7-[(3aα,4β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäure werden mit 57,6 mg (0,6 mMol) Methansulfonsäure in 5 ml Methanol fünf Minuten unter Rückfluß gekocht. Man läßt über Nacht nachrühren entfernt das Methanol im Vakuum und verrührt das Produkt mit Ethanol.
Ausbeute: 270 mg (90% der Theorie)
Schmelzpunkt: 220-222°C (unter Zersetzung)

### Beispiel 19:

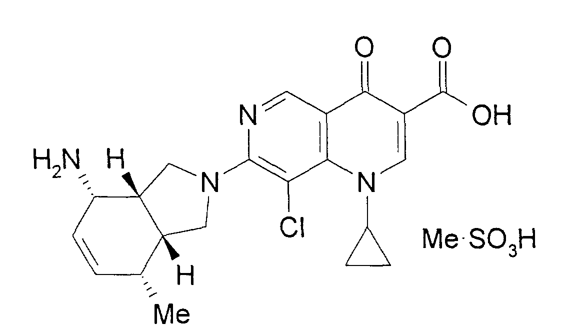

### 7-[(3aα,4β,7β,7aα)-4-Amino-1,3,3a,4,7,7a-hexahydroisoindol-2-yl]-8-chlor-1-cyclopropyl-1,4-dihydro-4-oxo-1,6-naphthyridin-3-carbonsäuremesylat

Analog zum Beispiel 18 wirdl bei der Umsetzung von 7-[(3aα,4β,7β,7aα)-4-Amino-7-methyl-1,3,3a,4,7,7a-hexahydroisoindol-2-yl]-8-chlor-1-cyclopropyl-1,4-dihydro-4-oxo-1,6-naphthyridin-3-carbonsäure die Titelverbindung erhalten.
Schmelzpunkt: 218-220°C (unter Zersetzung)

### Beispiel 20:

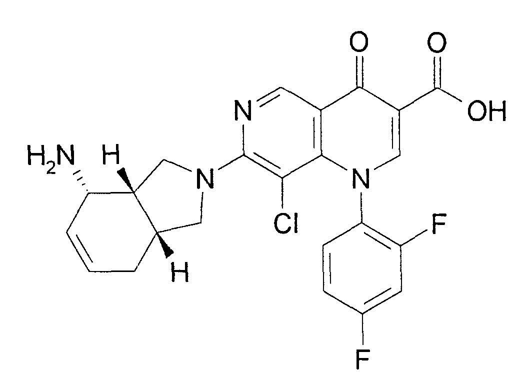

### 7-[(3aα,4β,7aα)-4-Amino-1,3,3a,4,7,7a-hexahydroisoindol-2-yl]-8-chlor-1-(2,4-difluorphenyl)-1,4-dihydro-4-oxo-1,6-naphthyridin-3-carbonsäure

372 mg (1 mMol) 7,8-Dichlor-1-(2,4-difluorphenyl)-1,4-dihydro-4-oxo-1,6-naphthyridin-3-carbonsäure werden mit 300 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff über Nacht bei Raumtemperatur gerührt. Der Niederschlag wird isoliert und mit Acetonitril verrührt.
Ausbeute: 400 mg (85% der Theorie)
Schmelzpunkt: 262-263°C (unter Zersetzung)

### Beispiel 21:

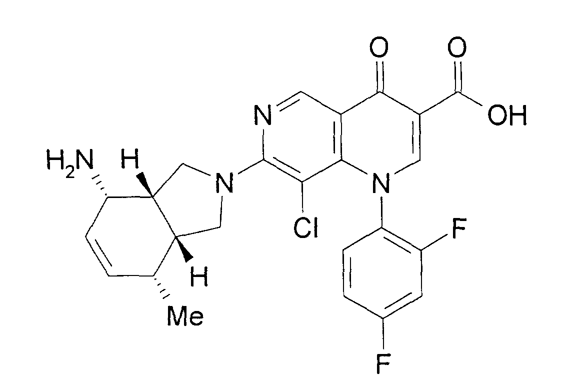

### 7-[(3aα,,4β,7β,7aα)-4-Amino-7-methyl-1,3,3a,4,7,7a-hexahydroisoindol-2-yl)-8-chlor-1-(2,4-difluorphenyl)-1,4-dihydro-4-oxo-1,6-naphthyridin-3-carbonsäure

372 mg (1 mMol) 7,8-Dichlor-1-(2,4-difluorphenyl)-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff vier Stunden bei 60°C gerührt. Der Ansatz wird mit Wasser versetzt, der Niederschlag isoliert und mit Acetonitril verrührt.
Ausbeute: 400 mg (86% der Theorie)
Schmelzpunkt: 258-260°C (unter Zersetzung)

### Beispiel 22:

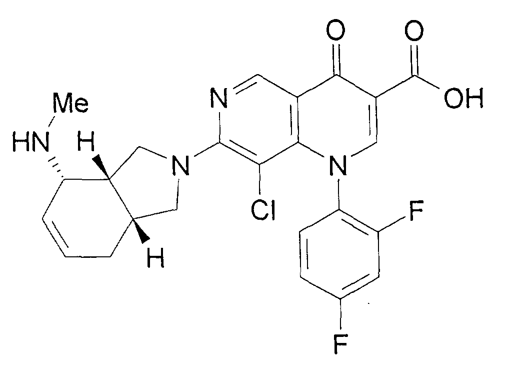

### 8-Chlor-1-(2,4-difluorphenyl)-1,4-dihydro-7-[(3aα,4β,7aα)-4-methylamino-1,3,3a,4,7,7a-hexahydroisoindol-2-yl]-4-oxo-1,6-naphthyridin-3-carbonsäure

372 mg (1 mMol) 7,8-Dichlor-1-(2,4-difluorphenyl)-1,4-dihydro-4-oxo-1,6-naphthyridin-3-carbonsäure werden mit 330 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril vier Stunden unter Stickstoff bei 60°C gerührt. Der Niederschlag wird isoliert und mit Acetonitril gewaschen.
Ausbeute: 420 mg (86% der Theorie)
Schmelzpunkt: 254-256°C (unter Zersetzung)

### Beispiel 23:

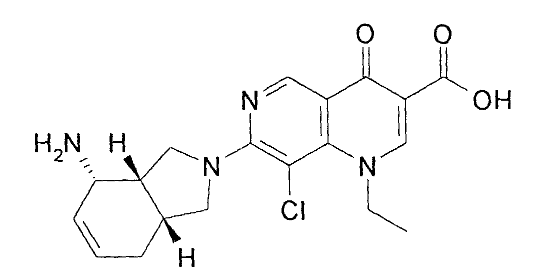

### 7-[(3aα,4β,7aα)-4-Amino-1,3,3a,4,7,7a-hexahydroisoindol-2-yl]-8-chlor-1-ethyl-1,4-dihydro-4-oxo-1,6-naphthyridin-3-carbonsäure

287 mg (1 mMol) 7,8-Dichlor-1-ethyl-1,4-dihydro-4-oxo-1,6-naphthyridin-3-carbonsäure werden mit 300 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff sechs Stunden bei 60°C gerührt. Es wird mit Eiswasser versetzt, der Niederschlag isoliert und mit Acetonitril verrührt.
Ausbeute: 340 mg (87% der Theorie)
Schmelzpunkt: 244-245°C (unter Zersetzung)

### Beispiel 24:

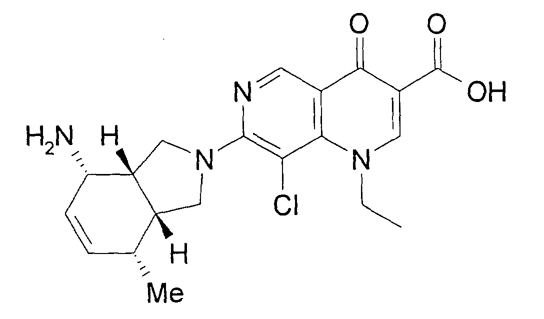

### 7-[(3aα,4β,7β,7aα)-4-Amino-7-methyl-1,3,3a,4,7,7a-hexahydroisoindol-2-yl]-8-chlor-1-ethyl-1,4-dihydro-4-oxo-1,6-naphthyridin-3-carbonsäure

287 mg (1 mMol) 7,8-Dichlor-1-ethyl-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff sechs Stunden bei 60°C gerührt. Der Ansatz wird mit Wasser versetzt, der Niederschlag isoliert und mit Acetonitril verrührt.
Ausbeute: 360 mg (89% der Theorie)
Schmelzpunkt: 231-233°C (unter Zersetzung)

### Beispiel 25:

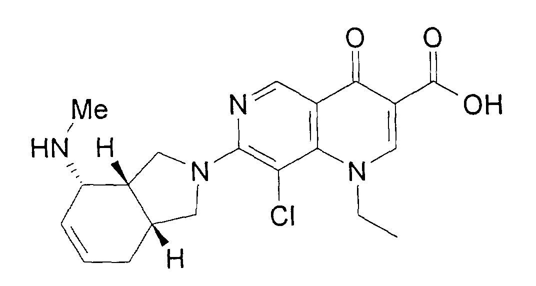

### 8-Chlor-1-ethyl-1,4-dihydro-7-[(3α,4β,7aα)-4-methylamino-1,3,3a,4,7,7a-hexahydroisoindol-2-yl]-4-oxo-1,6-naphthyridin-3-carbonsäure

287 mg (1 mMol) 7,8-Dichior-1-ethyl-1,4-dihydro-4-oxo-1,6-naphthyridin-3-carbonsäure werden mit 330 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril zwei Stunden unter Stickstoff bei 60°C gerührt. Der Niederschlag wird isoliert und mit Acetonitril gewaschen.
Ausbeute: 380 mg (94% der Theorie)
Schmelzpunkt: >300°C (unter Zersetzung)

### Beispiel 26:

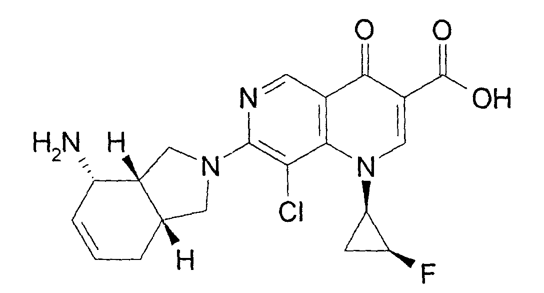

### 7-[(3aα,4β,7aα)-4-Amino-1,3,3a,4,7,7a-hexahydroisoindol-2-yl]-8-chlor-1-(cis-2-fluorcyclopropyl)-1,4-dihydro-4-oxo-1,6-naphthyridin-3-carbonsäure

317 mg (1 mMol) 7,8-Dichlor-1-(cis-2-fluorcyclopropyl)-1,4-dihydro-4-oxo-1,6-naphthyridin-3-carbonsäure werden mit 300 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff zwei Stunden bei 60°C gerührt. Es wird mit Eiswasser versetzt, der pH-Wert mit verdünnter Salzsäure auf 7 gestellt, der Niederschlag isoliert und mit Acetonitril verrührt.
Ausbeute: 310 mg (74% der Theorie)
Schmelzpunkt: 229-230°C (unter Zersetzung)

### Beispiel 27:

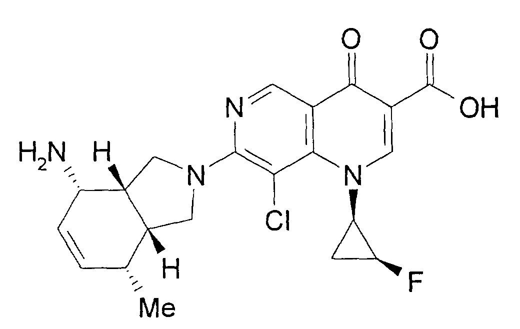

### 7-[(3aα,4β,7β,7aα)-4-Amino-7-methyl-1,3,3a,4,7,7a-hexahydroisoindol-2-yl]-8-chlor-1-(cis-2-fluor-cyclopropyl)-1,4-dihydro-4-oxo-1,6-naphthyridin-3-carbonsäure

317 mg (1 mMol) 7,8-Dichlor-1-(cis-2-fluorcyclopropyl)-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff sechs Stunden bei 60°C gerührt. Der Ansatz wird mit Wasser versetzt, der Niederschlag isoliert und mit Acetonitril verrührt.
Ausbeute: 330 mg (76% der Theorie)
Schmelzpunkt: 206-208°C (unter Zersetzung)

### Beispiel 28:

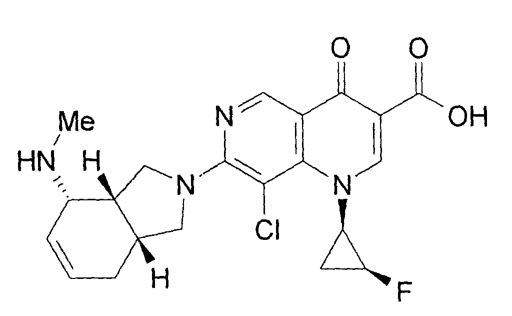

### 8-Chlor-1-(cis-2-fluorcyclopropyl)-1,4-dihydro-7-[(3aα,4β,7aα)-4-methylamino-1,3,3a,4,7,7a-hexahydroisoindol-2-yl]-4-oxo-1,6-naphthyridin-3-carbonsäure

317 mg (1 mMol) 7,8-Dichlor-1-(cis-2-fluorcyclopropyl)-1,4-dihydro-4-oxo-1,6-naphthyridin-3-carbonsäure werden mit 330 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril zwei Stunden unter Stickstoff bei 60°C gerührt. Der Niederschlag wird isoliert und mit Acetonitril gewaschen.
Ausbeute: 370 mg (85% der Theorie)
Schmelzpunkt: 199-200°C (unter Zersetzung)

### Beispiel 29:

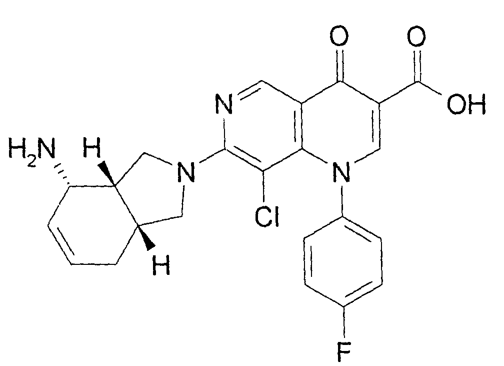

### 7-[(3aα,4β,7aα)-4-Amino-1,3,3a,4,7,7a-hexahydroisoindol-2-yl)-8-chlor-1-(4-fluorphenyl)-1,4-dihydro-4-oxo-1,6-naphthyridin-3-carbonsäure

353 mg (1 mMol) 7,8-Dichlor-1-(4-fluorphenyl)-1,4-dihydro-4-oxo-1,6-naphthyridin-3-carbonsäure werden mit 300 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff zehn Stunden bei 60°C gerührt. Es wird mit Eiswasser versetzt, der Niederschlag isoliert und mit Acetonitril verrührt.
Ausbeute: 410 mg (90% der Theorie)
Schmelzpunkt: 279-280°C (unter Zersetzung)

### Beispiel 30:

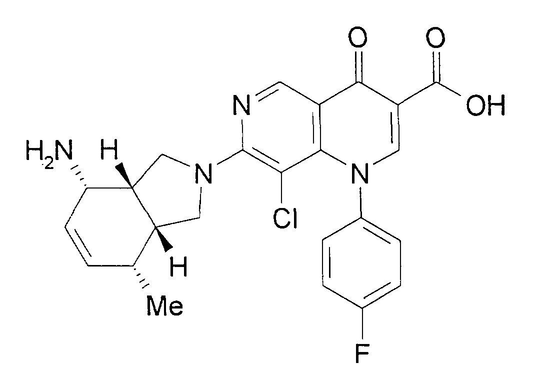

### 7-[(3aα,4β,7β,7aα)-4-Amino-7-methyl-1,3,3a,4,7,7a-hexahydroisoindol-2-yl)-8-chlor-1-(4-fluorphenyl)-1,4-dihydro-4-oxo-1,6-naphthyridin-3-carbonsäure

353 mg (1 mMol) 7,8-Dichlor-1-(4-fluorphenyl)-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff 16 Stunden bei 80°C gerührt. Der Ansatz wird mit Wasser versetzt, der Niederschlag isoliert und mit Acetonitril verrührt.
Ausbeute: 400 mg (86% der Theorie)
Schmelzpunkt: 251-253°C (unter Zersetzung)

### Beispiel 31:

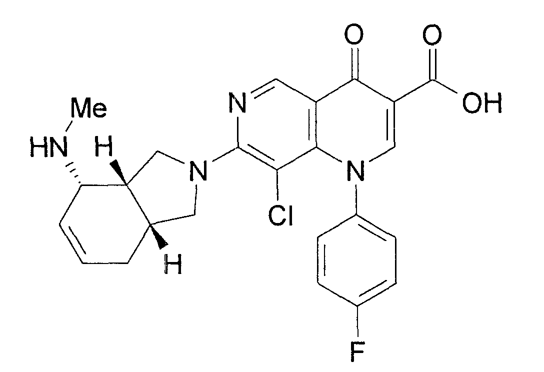

### 8-Chlor-1-(4-fluorphenyl)-1,4-dihydro-7-[(3aα,4β,7aα)-4-methylamino-1,3,3a,4,7,7a-hexahydroisoindol-2-yl]-4-oxo-1,6-naphthyridin-3-carbonsäure

353 mg (1 mMol) 7,8-Dichlor-1-(4-fluorphenyl)-1,4-dihydro-4-oxo-1,6-naphthyridin-3-carbonsäure werden mit 330 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril zehn Stunden unter Stickstoff bei 60°C gerührt. Der Niederschlag wird isoliert und mit Acetonitril gewaschen.
Ausbeute: 410 mg (88% der Theorie)
Schmelzpunkt: 238-240°C (unter Zersetzung)

### Beispiel 32:

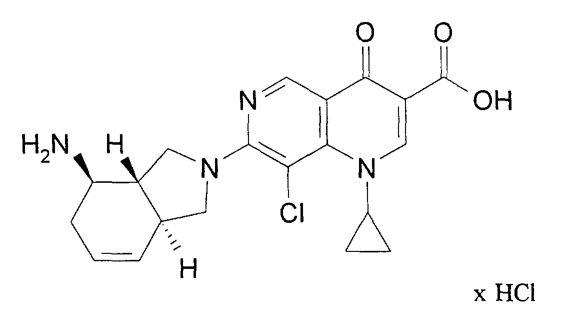

### 7-[(3aα,4α,7aβ)-4-Amino-1,3,3a,4,5,7a-hexahydroisoindol-2-yl]-8-chlor-1-cyclopropyl-1,4-dihydro-4-oxo-1,6-naphthyridin-3-carbonsäurehydrochlorid

1,05 g (3,5 mMol) 7,8-Dichlor-1-cyclopropyl-1,4-dihydro-4-oxo-1,6-naphthyridin-3-carbonsäure werden mit 1,62 g (7,7 mMol) (3aα,4α,7aβ)-4-Ethoxycarbonylamino-1,3,3a,4,5,7a-hexahydroisoindol in einem Gemisch aus 10,5 ml Dimethylformamid und 10,5 ml Acetonitril unter Stickstoff acht Stunden bei 80°C gerührt. Nach dem Abkühlen wird der Niederschlag isoliert und mit Acetonitril verrührt.

Der Rückstand (1,2 g) wird in 25 ml 10%iger Kalilauge und 12,5 ml Ethylenglykol sechs Stunden auf 130°C erwärmt. Der abgekühlte Ansatz wird mit 25 ml Methanol versetzt und der pH-Wert mit verdünnter Salzsäure auf 4-5 gestellt. Der ausfallende Feststoff wird isoliert und erneut in einem Gemisch aus 17,5 ml Methanol und 17,5 ml Wasser aufgenommen. Der pH-Wert wird mit konzentrierter Salzsäure auf 2-3 gestellt, der Feststoff isoliert und mit Acetonitril verrührt.
Ausbeute: 600 mg (42% der Theorie)
Schmelzpunkt: 259-260°C (unter Zersetzung)

### Beispiel 33:

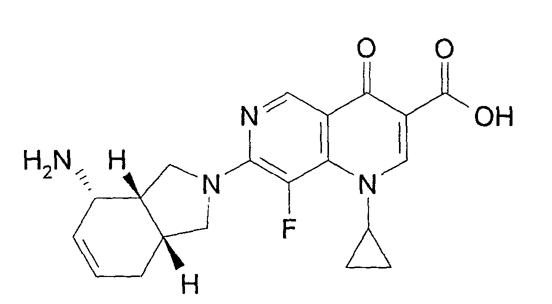

### 7-[(3aα,4β,7aα)-4-Amino-1,3,3a,4,7,7a-hexahydroisoindol-2-yl]-1-cyclopropyl-8-fluor-1,4-dihydro-4-oxo-1,6-naphthyridin-3-carbonsäure

282 mg (1 mMol) 7-Chlor-1-cyclopropyl-8-fluor-1,4-dihydro-4-oxo-1,6-naphthyridin-3-carbonsäure werden mit 303 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff sechs Stunden bei 80°C gerührt. Nach dem Abkühlen wird mit Eiswasser versetzt, der Niederschlag isoliert und mit Acetonitril verrührt.
Ausbeute: 340 mg (88% der Theorie)
Schmelzpunkt: 259-260°C (unter Zersetzung)

### Beispiel 34:

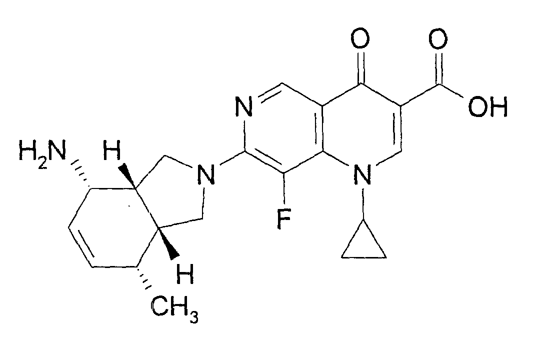

### 7-[(3aα,4β,7β,7aα)-4-Amino-7-methyl-1,3,3a,4,7,7a-hexahydroisoindol-2-yl]-1-cyclopropyl-8-fluor-1,4-dihydro-4-oxo-1,6-naphthyridin-3-carbonsäure

282 mg (1 mMol) 7-Chlor-1-cyclopropyl-8-fluor-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff sechs Stunden bei 80°C gerührt. Nach dem Abkühlen wird mit Eiswasser versetzt, der Niederschlag isoliert und mit Acetonitril verrührt.
Ausbeute: 310 mg (77% der Theorie)
Schmelzpunkt: 230-232°C (unter Zersetzung)

### Beispiel 35:

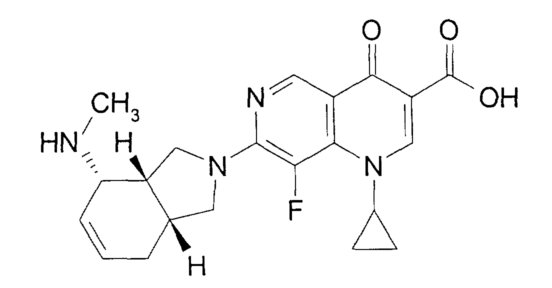

### 1-Cyclopropyl-8-fluor-1,4-dihydro7-[(3aα,4β,7aα)-4-methylamino-1,3,3a,4,7,7a-hexahydroisoindol-2-yl]-4-oxo-1,6-naphthyridin-3-carbonsäure

282 mg (1 mMol) 7-Chlor-1-cyclopropyl-8-fluor-1,4-dihydro-4-oxo-1,6-naphthyridin-3-carbonsäure werden mit 330 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril unter Stickstoff zwei Stunden bei 80°C gerührt. Nach dem Abkühlen wird mit Eiswasser versetzt, der Niederschlag isoliert und mit Acetonitril verrührt.
Ausbeute: 290 mg (72% der Theorie)
Schmelzpunkt: 231-233°C (unter Zersetzung)

### Beispiel 36:

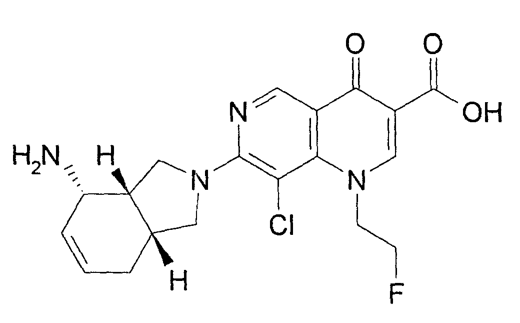

### 7-[(3aα,4β,7aα)-4-Amino-1,3,3a,4,7,7a-hexahydroisoindol-2-yl]-8-chlor-1-(2-fluorethyl)-1,4-dihydro-4-oxo-1,6-naphthyridin-3-carbonsäure

305 mg (1 mMol) 7,8-Dichlor-1-(2-fluorethyl)-1,4-dihydro-4-oxo-1,6-naphthyridin-3-carbonsäure werden mit 303 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 340 mg (83% der Theorie)
Schmelzpunkt: 241-242°C (unter Zersetzung)

### Beispiel 37:

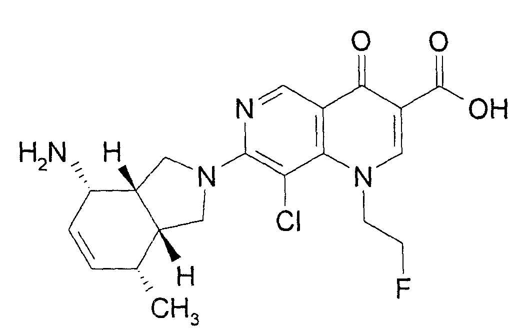

### 7-[(3aα,4β,7β,7aα)-4-Amino-7-methyl-1,3,3a,4,7,7a-hexahydroisoindol-2-yl]-8-chlor-1-(2-fluorethyl)-1,4-dihydro-4-oxo-1,6-naphthyridin-3-carbonsäure

305 mg (1 mMol) 7,8-Dichlor-1-(2-fluorethyl)-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 310 mg (73% der Theorie)
Schmelzpunkt: 212-214°C (unter Zersetzung)

### Beispiel 38:

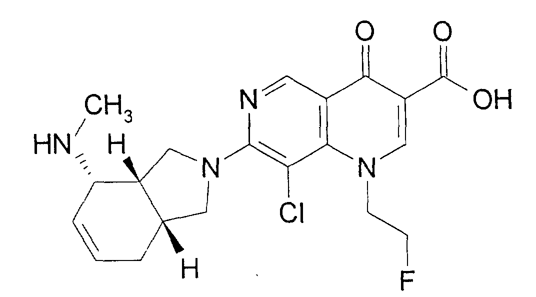

### 8-Chlor-1-(2-fluorethyl)-1,4-dihydro-7-[(3aα,4β,7aα)-4-methylamino-1,3,3a,4,7,7a-hexahydroisoindol-2-yl]-4-oxo-1,6-naphthyridin-3-carbonsäure

305 mg (1 mMol) 7,8-Dichlor-1-(2-fluorethyl)-1,4-dihydro-4-oxo-1,6-naphthyridin-3-carbonsäure werden mit 330 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 340 mg (80% der Theorie)
Schmelzpunkt: 220-221°C (unter Zersetzung)

### Beispiel 39:

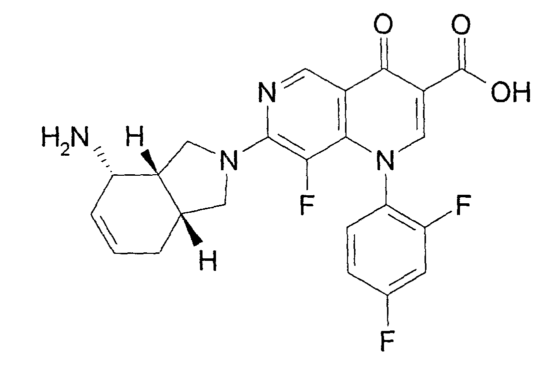

### 7-[(3aα,4β,7aα)-4-Amino-1,3,3a,4,7,7a-hexahydroisoindol-2-yl]-8-fluor-1-(2,4-difluorphenyl)-1,4-dihydro-4-oxo-1,6-naphthyridin-3-carbonsäure

354 mg (1 mMol) 7-Chlor-8-fluor-1-(2,4-difluorphenyl)-1,4-dihydro-4-oxo-1,6-naphthyridin-3-carbonsäure werden mit 303 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 380 mg (83% der Theorie)
Schmelzpunkt: 261-263°C (unter Zersetzung)

### Beispiel 40:

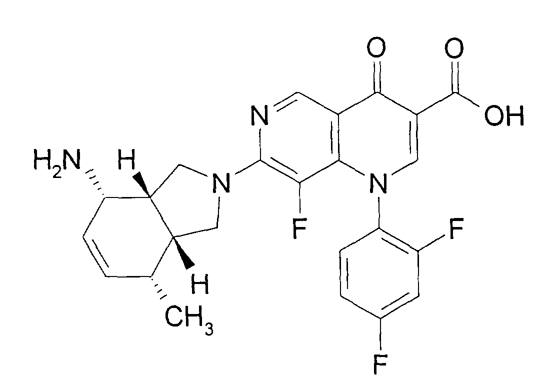

### 7-[(3aα,4β,7β,7aα)-4-Amino-7-methyl-1,3,3a,4,7,7a-hexahydroisoindol-2-yl]-8-fluor-1-(2,4-difluorphenyl)-1,4-dihydro-4-oxo-1,6-naphthyridin-3-carbonsäure

354 mg (1 mMol) 7-Chlor-8-fluor-1-(2,4-difluorphenyl)-1,4-dihydro-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 410 mg (87% der Theorie)
Schmelzpunkt: 158-162°C (unter Zersetzung)

### Beispiel 41:

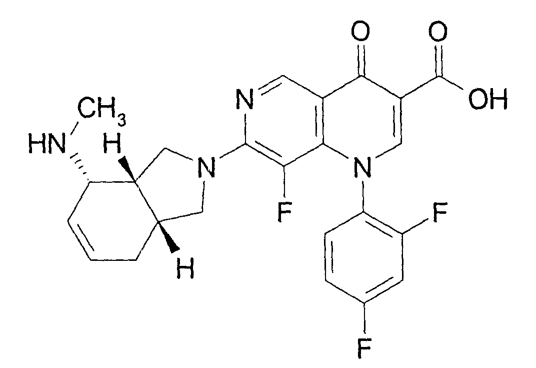

### 8-Fluor-1-(2,4-difluorphenyl)-1,4-dihydro-7-[(3aα,4β,7aα)-4-methylamino-1,3,3a,4,7,7a-hexahydroisoindol-2-yl]-4-oxo-1,6-naphthyridin-3-carbonsäure

177 mg (0,5 mMol) 7-Chlor-8-fluor-1-(2,4-difluorphenyl)-1,4-dihydro-4-oxo-1,6-naphthyridin-3-carbonsäure werden mit 167 mg (1,1 mMol) (3aα,4β,7aα)-4-Methylamino-1,3,3a,4,7,7a-hexahydroisoindol in einem Gemisch aus 1,5 ml Dimethylformamid und 1,5 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 170 mg (72% der Theorie)
Schmelzpunkt: 209-210°C (unter Zersetzung)

### Beispiel 42:

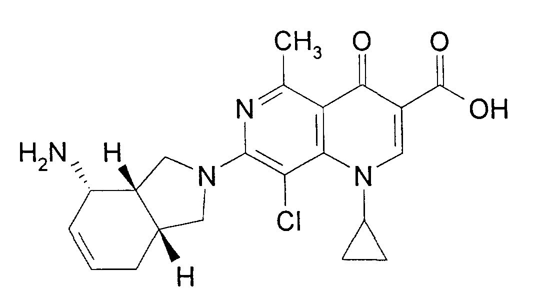

### 7-[(3aα,4β,7aα)-4-Amino-1,3,3a,4,7,7a-hexahydroisoindol-2-yl]-8-chlor-1-cyclopropyl-1,4-dihydro-5-methyl-4-oxo-1,6-naphthyridin-3-carbonsäure

313 mg (1 mMol) 7,8-Dichlor-1-cyclopropyl-1,4-dihydro-5-methyl-4-oxo-1,6-naphthyridin-3-carbonsäure werden mit 303 mg (2,2 mMol) (3aα,4β,7aα)-4-Amino-1,3,3a,4,7,7a-hexahydroisoindol in einem Gemisch aus 3 ml Dimethylformamid und 3 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 320 mg (77% der Theorie)
Schmelzpunkt: 225-227°C (unter Zersetzung)

### Beispiel 43:

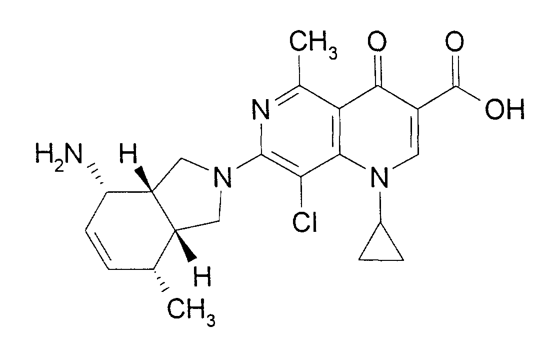

### 7-[(3aα,4β,7β,7aα)-4-Amino-7-methyl-1,3,3a,4,7,7a-hexahydroisoindol-2-yl]-8-chlor-1-cyclopropyl-1,4-dihydro-5-methyl-4-oxo-1,6-naphthyridin-3-carbonsäure

312 mg (1 mMol) 7,8-Dichlor-1-cyclopropyl-1,4-dihydro-5-methyl-4-oxo-1,6-naphthyridin-3-carbonsäure werden mit 330 mg (2,2 mMol) (3aα,4β,7β,7aα)-4-Amino-7-methyl-1,3,3a,4,7,7a-hexahydroisoindol in einem Gemisch aus 3 ml Dimethylformamid und 3 ml Acetonitril unter Stickstoff vier Stunden bei 80°C gerührt. Nach dem Abkühlen wird mit Eiswasser versetzt, der Niederschlag isoliert und mit Acetonitril verrührt.
Ausbeute: 320 mg (74% der Theorie)
Schmelzpunkt: 164-166°C (unter Zersetzung)

### Beispiel 44:

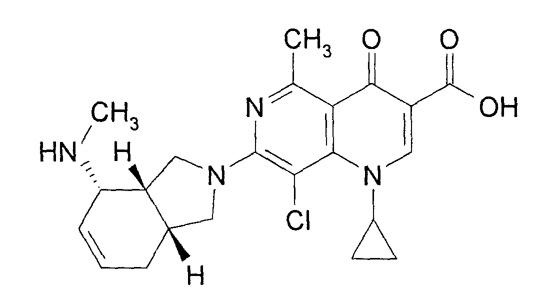

### 8-Chlor-1-cyclopropyl-1,4-dihydro-5-methyl-7-[(3aα,4β,7aα)-4-methylamino-1,3,3a,4,7,7a-hexahydroisoindol-2-yl]-4-oxo-1,6-naphthyridin-3-carbonsäure

313 mg (1 mMol) 7,8-Dichlor-1-cyclopropyl-1,4-dihydro-4-oxo-1,6-naphthyridin-3-carbonsäure werden mit 330 mg (2,2 mMol) (3aα,4β,7aα)-4-Methylamino-1,3,3a,4,7,7a-hexahydroisoindol in einem Gemisch aus 3 ml Dimethylformamid und 3 ml Acetonitril unter Stickstoff über Nacht bei Raumtemperatur gerührt. Anschließend wird mit Eiswasser versetzt, der Niederschlag isoliert und mit Acetonitril verrührt.
Ausbeute: 340 mg (79% der Theorie)
Schmelzpunkt: 165-167°C (unter Zersetzung)

### Beispiel 45:

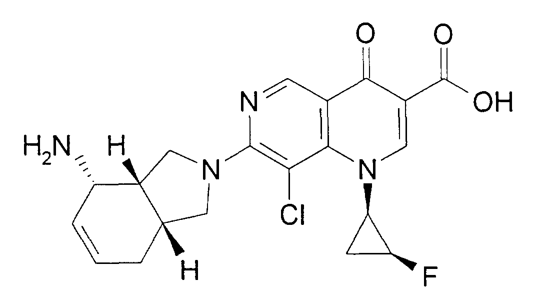

### 7-[(3aα,4β,7aα)-4-Amino-1,3,3a,4,7,7a-hexahydroisoindol-2-yl)-8-chlor-1-[(1R,2S)-2-fluorcyclopropyl]-1,4-dihydro-4-oxo-1,6-naphthyridin-3-carbonsäure

238 mg (0,75 mMol) 7,8-Dichlor-1-[(1R,2S)-2-fluorcyclopropyl]-1,4-dihydro-4-oxo-1,6-naphthyridin-3-carbonsäure werden mit 230 mg (1,65 mMol) (3aα,4β,7aα)-4-Amino-1,3,3a,4,7,7a-hexahydroisoindol in einem Gemisch aus 2,2 ml Dimethylformamid und 2,2 ml Acetonitril unter Stickstoff über Nacht bei Raumtemperatur gerührt. Anschließend wird mit Eiswasser versetzt, der Niederschlag isoliert und mit Acetonitril verrührt.
Ausbeute: 230 mg (71% der Theorie)
Schmelzpunkt: 224-226°C (unter Zersetzung)

### Beispiel 46:

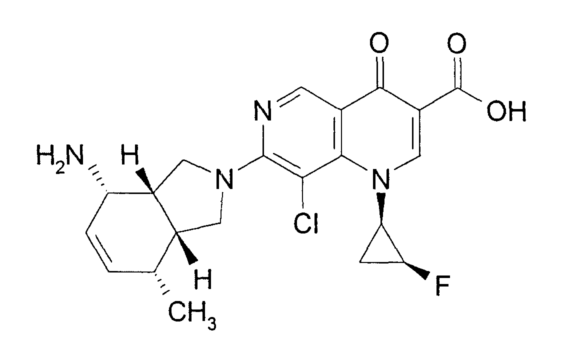

### 7-[(3aα,4β,7β,7aα)-4-Amino-7-methyl-1,3,3a,4,7,7a-hexahydroisoindol-2-yl]-8-chlor-1-[(1R,2S)-2-fluorcyclopropyl)-1,4-dihydro-5-methyl-4-oxo-1,6-naphthyridin-3-carbonsäure

238 mg (0,75 mMol) 7,8-Dichlor-1-[(1R,2S)-2-fluorcyclopropyl]-1,4-dihydro-4-oxo-1,6-naphthyridin-3-carbonsäure werden mit 250 mg (1,65 mMol) (3aα,4β,7β,7aα)-4-Amino-7-methyl-1,3,3a,4,7,7a-hexahydroisoindol in einem Gemisch aus 2,2 ml Dimethylformamid und 2,2 ml Acetonitril unter Stickstoff drei Stunden bei 60°C gerührt. Die Lösungsmittel werden im Vakuum entfernt, mit Eiswasser versetzt, der Niederschlag isoliert und mit Acetonitril verrührt.
Ausbeute: 190 mg (59% der Theorie)
Schmelzpunkt: 208-210°C (unter Zersetzung)

### Beispiel 47:

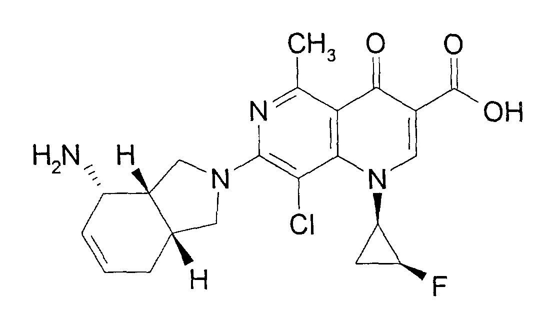

### 7-[(3aα,4β,7aα)-4-Amino-1,3,3a,4,7,7a-hexahydroisoindol-2-yl]-8-chlor-1-[(1R,2S)-2-fluorcyclopropyl)-1,4-dihydro-5-methyl-4-oxo-1,6-naphthyridin-3-carbonsäure

248 mg (0,75 mMol) 7,8-Dichlor-1-[(1R,2S)-2-fluorcyclopropyl]-1,4-dihydro-5-methyl-4-oxo-1,6-naphthyridin-3-carbonsäure werden mit 230 mg (1,65 mMol) (3aα,4β,7aα)-4-Amino-1,3,3a,4,7,7a-hexahydroisoindol in einem Gemisch aus 2,2 ml Dimethylformamid und 2,2 ml Acetonitril unter Stickstoff vier Stunden bei 60°C gerührt. Die Lösungsmittel werden im Vakuum entfernt, der Rückstand mit Eiswasser versetzt, der Niederschlag isoliert und mit Acetonitril verrührt.
Ausbeute: 210 mg (65% der Theorie)
Schmelzpunkt: 164-166°C (unter Zersetzung)

### Beispiel 48:

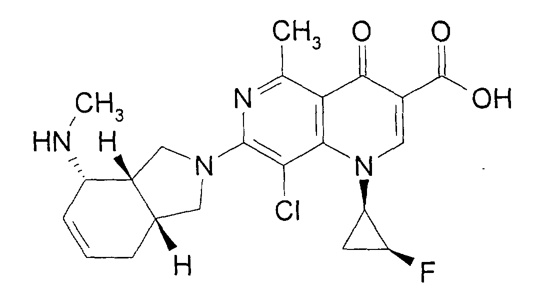

### 8-Chlor-1-[(1R,2S)-2-fluorcyclopropyl]-1,4-dihydro-5-methyl-7-[(3aα,4β,7aα)-4-methylamino-1,3,3a,4,7,7a-hexahydroisoindol-2-yl]-4-oxo-1,6-naphthyridin-3-carbonsäure

496 mg (1,5 mMol) 7,8-Dichlor-1-[(1R,2S)-2-fluorcyclopropyl]-1,4-dihydro-5-methyl-4-oxo-1,6-naphthyridin-3-carbonsäure werden mit 500 mg (3,3 mMol) (3aα,4β,7aα)-4-Methylamino-1,3,3a,4,7,7a-hexahydroisoindol in einem Gemisch aus 4,4 ml Dimethylformamid und 4,4 ml Acetonitril unter Stickstoff zwei Tage bei Raumtemperatur gerührt. Die Lösungsmittel werden im Vakuum entfernt, mit Eiswasser versetzt, der pH-Wert mit verdünnter Salzsäure auf 7 gestellt, der Niederschlag isoliert und mit Acetonitril verrührt.
Ausbeute: 550 mg (82% der Theorie)
Schmelzpunkt: 150-152°C (unter Zersetzung)

### Beispiel 49:

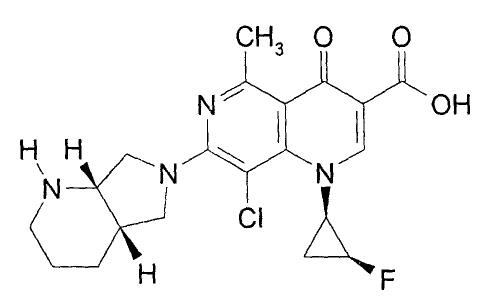

### 8-Chlor-1-[(1R,2S)-2-fluorcyclopropyl]-1,4-dihydro-7-[(1S,6S)-2,8-diazabicyclo[4.3.0]nonan-8-yl]-4-oxo-1,6-naphthyridin-3-carbonsäure

496 mg (1,5 mMol) 7,8-Dichlor-1-[(1R,2S)-2-fluorcyclopropyl]-1,4-dihydro-5-methyl-4-oxo-1,6-naphthyridin-3-carbonsäure werden mit 420 mg (1,5 mMol) (1S,6S)-2,8-Diazabicyclo[4.3.0]nonan in einem Gemisch aus 4,4 ml Dimethylformamid und 4,4 ml Acetonitril unter Argon zwei Tage bei Raumtemperatur gerührt. Die Lösungsmittel werden im Vakuum entfernt, der Rückstand mit Wasser versetzt, der pH-Wert mit verdünnter Salzsäure auf 7 gestellt, mit Dichlormethan extrahiert, über Natriumsulfat getrocknet und einrotiert.
Ausbeute: 550 mg (87% der Theorie)
Schmelzpunkt: 147-149°C (unter Zersetzung)

### Beispiel 50:

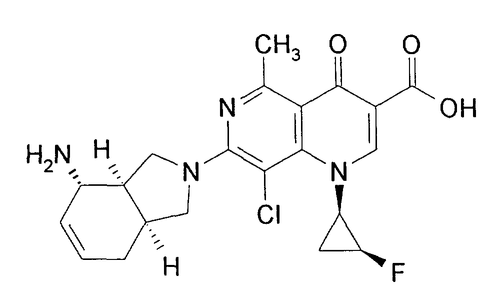

### 7-[(3aα,4α,7aα)-4-Amino-1,3,3a,4,7,7a-hexahydroisoindol-2-yl]-8-chlor-1-[(1R,2S)-2-fluorcyclopropyl]-1,4-dihydro-5-methyl-4-oxo-1,6-naphthyridin-3-carbonsäure

248 mg (0,75 mMol) 7,8-Dichlor-1-[(1R,2S)-2-fluorcyclopropyl)-1,4-dihydro-5-methyl-4-oxo-1,6-naphthyridin-3-carbonsäure werden mit 230 mg (1,65 mMol) (3aα,4α,7aα)-4-Amino-1,3,3a,4,7,7a-hexahydroisoindol in einem Gemisch aus 2,2 ml Dimethylformamid und 2,2 ml Acetonitril unter Stickstoff zwei Stunden bei 60°C gerührt. Die Lösungsmittel werden im Vakuum entfernt, mit Eiswasser versetzt, der pH-Wert mit verdünnter Salzsäure auf 7 gestellt, der Niederschlag isoliert und mit Acetonitril verrührt.
Ausbeute: 298 mg (90 % der Theorie)
Schmelzpunkt: 179-180°C (unter Zersetzung)

## Patentansprüche

1. Verbindungen der allgemeinen Formel (I) 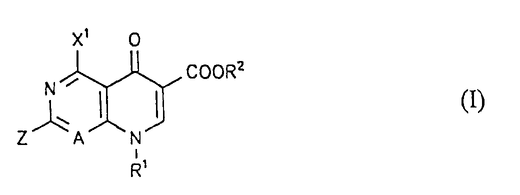 in welcher
R¹ für gegebenenfalls durch Hydroxy, Halogen oder C₁-C₃-Alkoxy substituiertes geradkettiges oder verzweigtes C₁-C₄-Alkyl, für gegebenenfalls durch C₁-C₃-Alkyl oder Halogen substituiertes C₃-C₆-Cycloalkyl, C₂-C₄-Alkenyl, ferner für C₁-C₃-Alkoxy, Amino, Monoalkylamino mit 1 bis 3 C-Atomen, Dialkylamino mit 1 bis 3 C-Atomen je Alkylgruppe oder für gegebenenfalls durch Halogen ein- bis dreifach substituiertes Phenyl steht,
R² für Wasserstoff, gegebenenfalls durch Hydroxy, Methoxy, Amino, Methylamino, oder Dimethylamino substituiertes Alkyl mit 1 bis 4 Kohlenstoffatomen oder (5-Methyl-2-oxo-1,3-dioxol-4-yl)-methyl,
X¹ für Wasserstoff, Halogen, Amino, Methyl oder Trifluormethyl,
Z für Reste der Strukturen 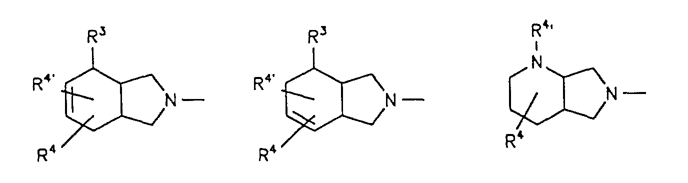 worin
R³ für Wasserstoff, Hydroxy, -NR⁵R⁶, Hydroxymethyl oder-CH₂-NR⁵R⁶ steht,
wobei
R⁵ Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl und
R⁶ Wasserstoff oder Methyl bedeutet,
R⁴ für Wasserstoff, geradkettiges oder verzweigtes C₁-C₃-Alkyl oder Cyclopropyl,
R^{4'} für Wasserstoff oder Methyl,
A für C-R⁷ steht, worin
R⁷ für Wasserstoff, Halogen, Methyl, Trifluormethyl, Vinyl, Ethinyl, Hydroxy oder Methoxy steht oder auch gemeinsam mit R¹ eine Brücke der Struktur 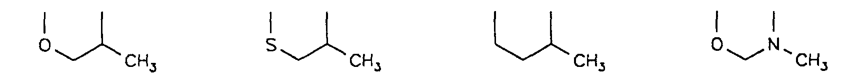
bilden kann, in Form von Racematen oder als enantiomerenreine Verbindungen, deren pharmazeutisch verwendbare Hydrate und Säureadditionsalze sowie die Alkali-, Erdalkali-, Silber- und Guanidiniumsalze der zugrundeliegenden Naphthyridonsäuren.

2. Verbindungen gemäß Anspruch 1, Formel (I),
in welcher
R¹ für gegebenenfalls durch Hydroxy oder Fluor substituiertes C₁-C₂-Alkyl, für gegebenenfalls durch Fluor substituiertes C₃-C₅-Cycloalkyl, Vinyl, Amino, Monoalkylamino mit 1 bis 2 C-Atomen, Dialkylamino mit 1 bis 2 C-Atomen je Alkylgruppe oder für gegebenenfalls durch Halogen ein- bis zweifach substituiertes Phenyl steht,
R² für Wasserstoff, gegebenenfalls durch Amino oder Dimethylamino substituiertes Alkyl mit 1 bis 2 Kohlenstoffatomen oder (5-Methyl-2-oxo-1,3-dioxol-4-yl)-methyl,
X¹ für Wasserstoff, Fluor, Chlor, Amino, Methyl oder Trifluormethyl,
Z für Reste der Strukturen 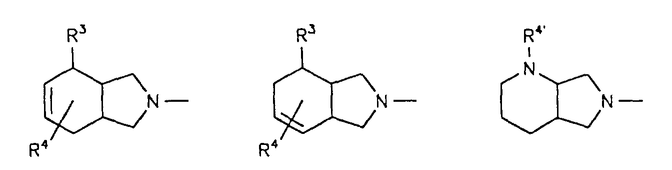 worin
R³ für Wasserstoff, Hydroxy, -NR⁵R⁶, Hydroxymethyl oder-CH₂-NR⁵R⁶ steht,
wobei
R⁵ Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl und
R⁶ Wasserstoff oder Methyl bedeutet,
R⁴ für Wasserstoff, geradkettiges oder verzweigtes C₁-C₃-Alkyl oder Cyclopropyl,
R^{4'} für Wasserstoff oder Methyl,
A für C-R⁷ steht, worin
R⁷ für Wasserstoff, Chlor, Fluor, Methyl, Trifluormethyl, Hydroxy oder Methoxy steht oder auch gemeinsam mit R¹ eine Brücke der Struktur bilden kann, 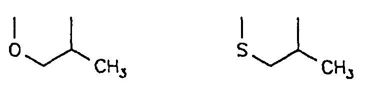
und deren pharmazeutisch verwendbare Hydrate und Säureadditionsalze sowie die Alkali-, Erdalkali-, Silber- und Guanidiniumsalze der zugrundeliegenden Naphthyridonsäuren.

3. Verbindungen der Formel (I) nach Anspruch 1, in welcher
R¹ für Methyl, Ethyl, gegebenenfalls durch Fluor substituiertes Cyclopropyl oder für gegebenenfalls durch Fluor ein- bis zweifach substituiertes Phenyl steht,
R² für Wasserstoff, Methyl oder Ethyl,
X¹ für Wasserstoff, Methyl oder Trifluormethyl,
Z für Reste der Strukturen 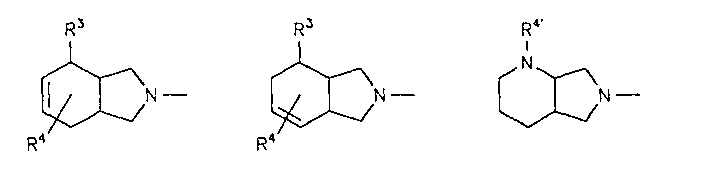 worin
R³ für Wasserstoff, Hydroxy, -NR⁵R⁶, Hydroxymethyl oder-CH₂-NR⁵R⁶ steht,
wobei
R⁵ Wasserstoff, Methyl, Alkoxycarbonyl mit 1 bis 4 C-Atomen im Alkoxyteil oder C₁-C₃-Acyl und
R⁶ Wasserstoff oder Methyl bedeutet,
R⁴ für Wasserstoff, geradkettiges oder verzweigtes C₁-C₃-Alkyl oder Cyclopropyl,
R^{4'} für Wasserstoff oder Methyl,
A für C-R⁷ steht, worin
R⁷ für Wasserstoff, Chlor, Fluor, Methoxy steht oder auch gemeinsam mit R¹ eine Brücke der Struktur 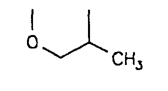
bilden kann,
und deren pharmazeutisch verwendbare Hydrate und Säureadditionsalze sowie die Alkali-, Erdalkali-, Silber- und Guanidiniumsalze der zugrundeliegenden Naphthyridonsäuren.

4. 8-Chlor-1-cyclopropyl-1,4-dihydro-5-methyl-7-[(3aα,4β,7aα)-4-methylamino-1,3,3a,4,7,7a-hexahydroisoindol-2-yl]-4-oxo-1,6-naphthyridin-3-carbonsäure der Formel 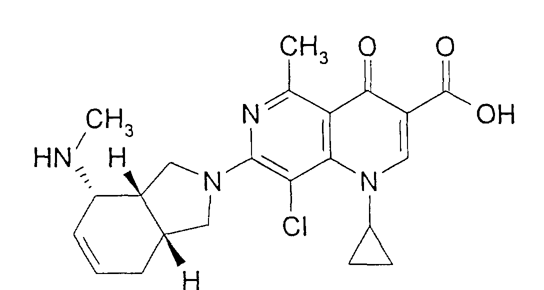 nach Anspruch 1 und dessen pharmazeutisch verwendbare Hydrate und Säureadditionssalze sowie die Alkali-, Erdalkali-, Silber- und Guanidiniumsalze.

5. Verfahren zur Herstellung von Verbindungen gemäß Anspruch 1, der Formel (I), dadurch gekennzeichnet, daß man Verbindungen der Formel (II) 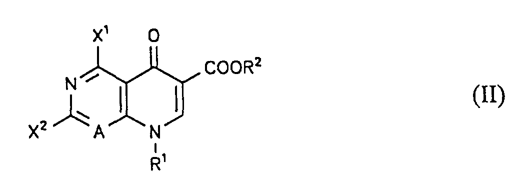 in welcher
R¹, R², X¹, und A die in Anspruch 1 angegebene Bedeutung haben und
X² für Halogen, insbesondere Fluor oder Chlor, steht,
mit Verbindungen der Formel (III)
Z-H (III)
in welcher
Z die in Anspruch 1 angegebene Bedeutung hat,
gegebenenfalls in Gegenwart von Säurefängern umsetzt.

6. Arzneimittel, enthaltend Verbindungen gemäß Ansprüchen 1 bis 4.

7. Antimikrobielle Mittel, enthaltend Verbindungen gemäß Ansprüchen 1 bis 4.

8. Verwendung von Verbindungen gemäß Ansprüchen 1 bis 4 zur Herstellung von Arzneimitteln zur Bekämpfung von Krankheiten.

9. Verwendung von Verbindungen gemäß Ansprüchen 1 bis 4 zur Herstellung von Arzneimitteln zur Bekämpfung von bakteriellen Infektionen.

## Claims

1. Compounds of the general formula (I) 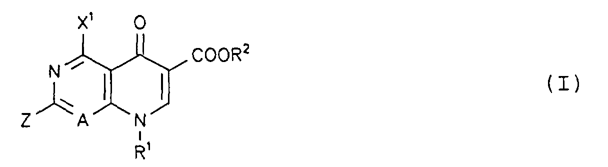 in which
R¹ represents straight-chain or branched C₁-C₄-alkyl which is optionally substituted by hydroxyl, halogen or C₁-C₃-alkoxy, or represents C₃-C₆-cycloalkyl which is optionally substituted by C₁-C₃-alkyl or halogen, or represents C₂-C₄-alkenyl, or furthermore represents C₁-C₃-alkoxy, amino, monoalkylamino having 1 to 3 carbon atoms, dialkylamino having 1 to 3 carbon atoms per alkyl group, or represents phenyl which is optionally substituted from one to three times by halogen,
R² represents hydrogen, alkyl having 1 to 4 carbon atoms which is optionally substituted by hydroxyl, methoxy, amino, methylamino or dimethylamino, or represents (5-methyl-2-oxo-1,3-dioxol-4-yl)-methyl,
X¹ represents hydrogen, halogen, amino, methyl or trifluoromethyl,
Z represents radicals of the structures 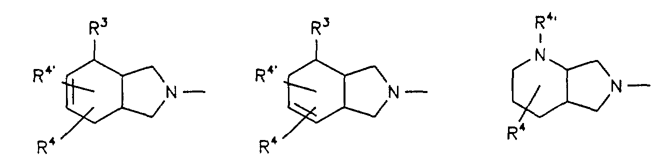 in which
R³ represents hydrogen, hydroxyl, -NR⁵R⁶, hydroxymethyl or -CH₂-NR⁵R⁶,
where
R⁵ denotes hydrogen, C₁-C₃-alkyl which is optionally substituted by hydroxyl, or denotes alkoxycarbonyl having 1 to 4 carbon atoms in the alkoxy moiety or denotes C₁-C₃-acyl, and
R⁶ denotes hydrogen or methyl,
R⁴ represents hydrogen, straight-chain or branched C₁-C₃-alkyl or cyclopropyl,
R^{4'} represents hydrogen or methyl,
A represents C-R⁷, in which
R⁷ represents hydrogen, halogen, methyl, trifluoromethyl, vinyl, ethinyl, hydroxyl or methoxy or else, together with R¹, can form a bridge of the structure 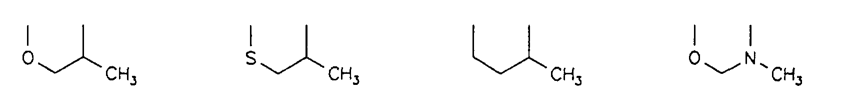
in the form of racemates or as enantiomerically pure compounds, pharmaceutically utilizable hydrates and acid addition salts thereof, and the alkali metal, alkaline earth metal, silver and guanidinium salts of the naphthyridonecarboxylic acids on which they are based.

2. Compounds according to Claim 1, formula (I),
in which
R¹ represents C₁-C₂-alkyl which is optionally substituted by hydroxyl or fluorine, or represents C₃-C₅-cycloalkyl which is optionally substituted by fluorine, or represents vinyl, amino, monoalkylamino having 1 to 2 carbon atoms, dialkylamino having 1 to 2 carbon atoms per alkyl group, or represents phenyl which is optionally substituted from one to two times by halogen,
R² represents hydrogen, alkyl having 1 to 2 carbon atoms which is optionally substituted by amino or dimethylamino, or represents (5-methyl-2-oxo-1,3-dioxol-4-yl)-methyl,
X¹ represents hydrogen, fluorine, chlorine, amino, methyl or trifluoromethyl,
Z represents radicals of the structures 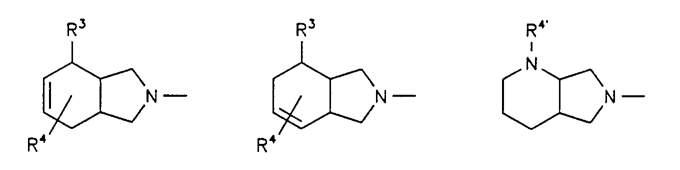 in which
R³ represents hydrogen, hydroxyl, -NR⁵R⁶, hydroxymethyl or -CH₂-NR⁵R⁶,
where
R⁵ denotes hydrogen, C₁-C₂-alkyl which is optionally substituted by hydroxyl, or denotes alkoxycarbonyl having 1 to 4 carbon atoms in the alkoxy moiety, or denotes C₁-C₃-acyl, and
R⁶ denotes hydrogen or methyl,
R⁴ represents hydrogen, straight-chain or branched C₁-C₃-alkyl or cyclopropyl,
R^{4'} represents hydrogen or methyl,
A represents C-R⁷, in which
R⁷ represents hydrogen, chlorine, fluorine, methyl, trifluoromethyl, hydroxyl or methoxy, or else, together with R¹, can form a bridge of the structure 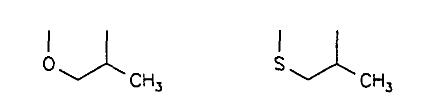
and the pharmaceutically utilizable hydrates and acid addition salts thereof, and also the alkali metal, alkaline earth metal, silver and guanidinium salts of the naphthyridonecarboxylic acids on which they are based.

3. Compounds of the formula (I) according to claim 1, in which
R¹ represents methyl, ethyl, cyclopropyl which is optionally substituted by fluorine, or represents phenyl which is optionally substituted from one to two times by fluorine,
R² represents hydrogen, methyl or ethyl,
X¹ represents hydrogen, methyl or trifluoromethyl,
Z represents radicals of the structures 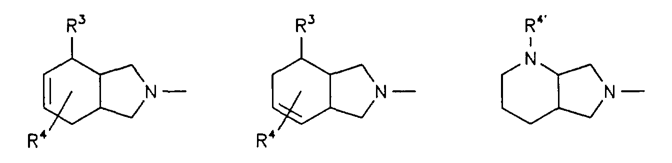 in which
R³ represents hydrogen, hydroxyl, -NR⁵R⁶, hydroxymethyl or -CH₂-NR⁵R⁶, where
R⁵ denotes hydrogen, methyl, alkoxycarbonyl having 1 to 4 carbon atoms in the alkoxy moiety, or denotes C₁-C₃-acyl, and
R⁶ denotes hydrogen or methyl,
R⁴ represents hydrogen, straight-chain or branched C₁-C₃-alkyl or cyclopropyl,
R^{4'} represents hydrogen or methyl,
A represents C-R⁷ in which
R⁷ represents hydrogen, chlorine, fluorine or methoxy, or else, together with R¹, can form a bridge of the structure 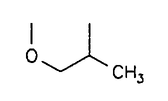
and the pharmaceutically utilizable hydrates and acid addition salts thereof, and also the alkali metal, alkaline earth metal, silver and guanidinium salts of the naphthyridonecarboxylic acid on which they are based.

4. 8-Chloro-1-cyclopropyl-1,4-dihydro-5-methyl-7-[(3aα,4β,7aα)-4-methylamino-1,3,3a,4,7,7a-hexahydroisoindol-2-yl]-4-oxo-1,6-naphthyridine-3-carboxylic acid of the formula 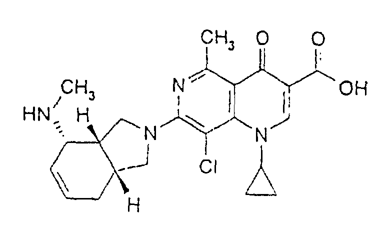 according to Claim 1 and its pharmaceutically utilizable hydrates and acid addition salts and the alkali metal, alkaline earth metal, silver and guanidinium salts.

5. Process for the preparation of compounds according to Claim 1, of the formula (I), characterized in that compounds of the formula (II) 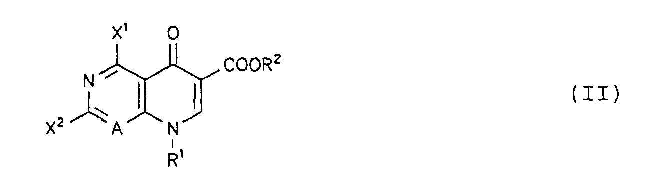 in which
R¹, R², X¹ and A have the meaning given in Claim 1, and
X² represents halogen, especially fluorine or chlorine, are reacted with compounds of the formula (III)
Z-H (III)
in which
Z has the meaning given in Claim 1,
optionally in the presence of acid scavengers.

6. Medicaments containing compounds according to Claims 1 to 4.

7. Antimicrobial compositions containing compounds according to Claims 1 to 4.

8. Use of compounds according to Claims 1 to 4 for preparing medicaments for the combating of diseases.

9. Use of compounds according to Claims 1 to 4 for preparing medicaments for the combating of bacterial infections.

## Revendications

1. Composés de formule générale (I) 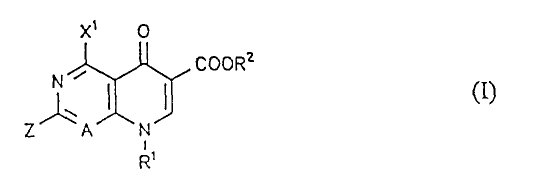 dans laquelle
R¹ représente un groupe alkyle en C₁ à C₄ linéaire ou ramifié portant éventuellement un substituant hydroxy, halogéno ou alkoxy en C₁ à C₃, un groupe cycloalkyle en C₃ à C₆ portant éventuellement un substituant alkyle en C₁ à C₃ ou halogéno, un groupe alcényle en C₂ à C₄, en outre un groupe alkoxy en C₁ à C₃, amino, mono-alkylamino ayant 1 à 3 atomes de carbone, dialkylamino ayant 1 à 3 atomes de carbone par groupe alkyle ou un groupe phényle portant éventuellement 1 à 3 substituants halogéno,
R² représente l'hydrogène, un groupe alkyle ayant 1 à 4 atomes de carbone portant éventuellement un substituant hydroxy, méthoxy, amino, méthylamino ou diméthylamino ou un groupe (5-méthyl-2-oxo-1,3-dioxole-4 yl)-méthyle,
X¹ représente l'hydrogène, un halogène, un groupe amino, méthyle ou trifluorométhyle,
Z représente des restes de structures 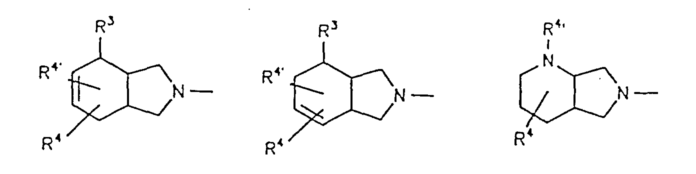 où
R³ représente l'hydrogène, un groupe hydroxy, -NR⁵R⁶, hydroxyméthyle ou -CH₂-NR⁵R⁶,
où
R⁵ représente l'hydrogène, un groupe alkyle en C₁ à C₃ portant éventuellement un substituant hydroxy, un groupe alkoxy-carbonyle ayant 1 à 4 atomes de carbone dans la partie alkoxy ou un groupe acyle en C₁ à C₃ et
R⁶ représente l'hydrogène ou un groupe méthyle,
R⁴ représente l'hydrogène, un groupe alkyle en C₁ à C₃ linéaire ou ramifié ou le groupe cyclopropyle,
R^{4'} représente l'hydrogène ou le groupe méthyle,
A est un groupe C-R⁷ dans lequel
R⁷ représente l'hydrogène, un halogène, un groupe méthyle, trifluorométhyle, vinyle, éthynyle, hydroxy ou méthoxy ou peut aussi former conjointement avec R¹ un pont de structure 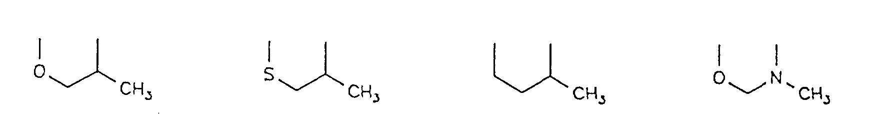
sous forme de racémates ou de composés énantiomères purs, leurs hydrates et sels d'addition d'acides acceptables du point de vue pharmaceutique ainsi que les sels de métaux alcalins, de métaux alcalino-terreux, d'argent et de guanidinium des acides naphtyridone-carboxyliques de base.

2. Composés suivant la revendication 1, de formule (I) dans laquelle
R¹ représente un groupe alkyle en C₁ ou C₂ éventuellement substitué par un groupe hydroxy ou du fluor,
un groupe cycloalkyle en C₃ à C₅ éventuellement substitué par du fluor, un groupe vinyle, amino, mono-alkylamino ayant 1 ou 2 atomes de carbone, dialkylamino ayant 1 ou 2 atomes de carbone par groupe alkyle ou un groupe phényle éventuellement substitué une ou deux fois par un halogène,
R² représente l'hydrogène, un groupe alkyle ayant 1 ou 2 atomes de carbone éventuellement substitué par un radical amino ou diméthylamino ou un groupe (5-méthyl-2-oxo-1,3-dioxole-4-yl)-méthyle,
X¹ représente l'hydrogène, le fluor, le chlore, un groupe amino, méthyle ou trifluorométhyle,
Z représente des restes de structures 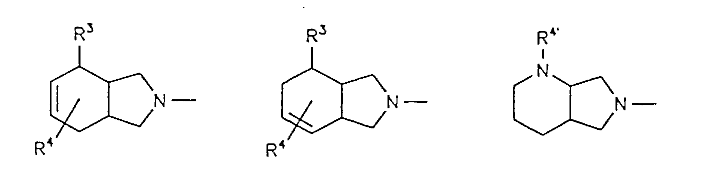 où
R³ représente l'hydrogène, un groupe hydroxy, -NR⁵R⁶, hydroxyméthyle ou -CH₂-NR⁵R⁶,
où
R⁵ représente l'hydrogène, un groupe alkyle en C₁ ou C₂ éventuellement substitué par un radical hydroxy, un groupe alkoxycarbonyle ayant 1 à 4 atomes de carbone dans la partie alkoxy ou un groupe acyle en C₁ à C₃ et
R⁶ représente l'hydrogène ou le groupe méthyle,
R⁴ représente l'hydrogène, un groupe alkyle en C₁ à C₃ linéaire ou ramifié ou le groupe cyclopropyle,
R^{4'} représente l'hydrogène ou le groupe méthyle,
A est un groupe C-R⁷ dans lequel
R⁷ représente l'hydrogène, le chlore, le fluor, un groupe méthyle, trifluorométhyle, hydroxy ou méthoxy ou peut aussi former conjointement avec R¹ un pont de structure 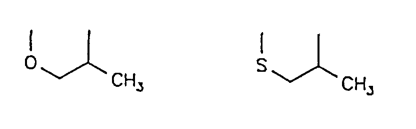
et leurs hydrates et sels d'addition d'acides acceptables du point de vue pharmaceutique ainsi que les sels de métaux alcalins, de métaux alcalino-terreux, d'argent et de guanidinium des acides naphtyridone-carboxyliques de base.

3. Composés de formule (I) suivant la revendication 1, dans laquelle
R¹ représente un groupe méthyle, éthyle, un groupe cyclopropyle éventuellement substitué par du fluor ou un groupe phényle éventuellement substitué une ou deux fois par du fluor,
R² représente l'hydrogène, un groupe méthyle ou éthyle
X¹ représente l'hydrogène, un groupe méthyle ou trifluorométhyle,
Z représente des restes de structures 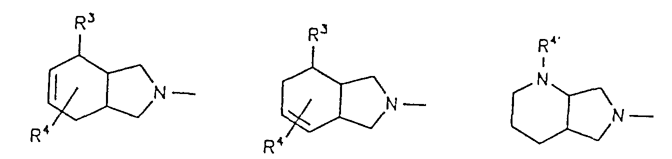 où
R³ représente l'hydrogène, un groupe hydroxy, -NR⁵R⁶, hydroxyméthyle ou -CH₂-NR⁵R⁶,
où
R⁵ représente l'hydrogène, un groupe méthyle, alkoxycarbonyle ayant 1 à 4 atomes de carbone dans la partie alkoxy ou acyle en C₁ à C₃ et
R⁶ représente l'hydrogène ou le groupe méthyle,
R⁴ représente l'hydrogène, un groupe alkyle en C₁ à C₃ linéaire ou ramifié ou le groupe cyclopropyle,
R^{4'} représente l'hydrogène ou le groupe méthyle,
A est un groupe C-R⁷ dans lequel
R⁷ représente l'hydrogène, le chlore, le fluor, le groupe méthoxy, ou peut aussi former conjointement avec R¹ un pont de structure 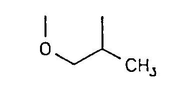
et leurs hydrates et sels d'addition d'acides acceptables du point de vue pharmaceutique ainsi que les sels de métaux alcalins, de métaux alcalino-terreux, d'argent et de guanidinium des acides naphtyridone-carboxyliques de base.

4. L'acide 8-chloro-1-cyclopropyl-1,4-dihydro-5-méthyl-7- [(3aα,4β,7aα)-4-méthylamino-1,3,3a,4,7,7a-hexahydro-iso-indole-2-yl] -4-oxo-1,6-naphtyridine-3-carboxylique de formule 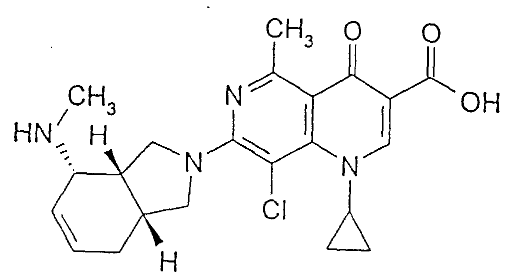 suivant la revendication 1 et ses hydrates et sels d'addition d'acides acceptables du point de vue pharmaceutique ainsi que les sels de métaux alcalins, de métaux alcalino-terreux, d'argent et de guanidinium.

5. Procédé de production de composés de formule (I) suivant la revendication 1, caractérisé en ce qu'on fait réagir des composés de formule (II) 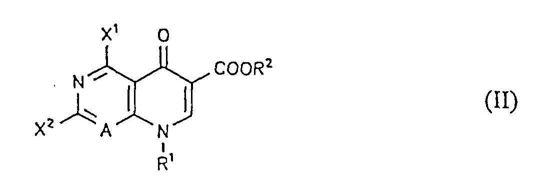 dans laquelle
R¹, R², X¹ et A ont la définition indiquée dans la revendication 1 et
X² représente un halogène, en particulier le fluor ou le chlore,
avec des composés de formule (III)
Z-H (III)
dans laquelle
Z a la définition indiquée dans la revendication 1, éventuellement en présence d'accepteurs d'acides.

6. Médicaments contenant des composés suivant les revendications 1 à 4.

7. Compositions antimicrobiennes contenant des composés suivant les revendications 1 à 4.

8. Utilisation de composés suivant les revendications 1 à 4 pour la préparation de médicaments destinés à combattre des maladies.

9. Utilisation de composés suivant les revendications 1 à 4 pour la préparation de médicaments destinés à combattre des infections bactériennes.
